# DEMANDE DE BREVET EUROPEEN

(11) **EP 4 151 749 A1**
(43) Date de publication de la demande: **22.03.2023**
(21) Numéro de dépôt: 22195865.5
(22) Date de dépôt: 15.09.2022
(51) Int. Cl.: C12Q 1/6804, C12Q 1/6844

(54) **OLIGONUCLÉOTIDE À DOUBLE STRUCTURE TIGE-BOUCLE ET UTILISATION DANS LA DÉTECTION D'ANALYTES D'INTÉRÊT**

(30) Priorité: 15.09.2021 FR 2109667
(71) Demandeur: Commissariat à l'énergie atomique et aux énergies alternatives, 75015 Paris (FR); CENTRE NATIONAL DE LA RECHERCHE SCIENTIFIQUE, 75794 Paris Cedex 16 (FR)
(72) Inventeur: AUBRET, Mathilde, 38054 GRENOBLE CEDEX 09 (FR); BOURDAT, Anne-Gaëlle, 38054 GRENOBLE CEDEX 09 (FR); BUHOT, Arnaud, 38054 GRENOBLE CEDEX 09 (FR); CUBIZOLLES, Myriam-Laure, 38054 GRENOBLE CEDEX 09 (FR); ROUPIOZ, Yoann, 38570 THEYS (FR); SAVONNET, Maud, 38054 GRENOBLE CEDEX 09 (FR)
(74) Mandataire: Brevalex

(57) **Abrégé**

La présente invention concerne un oligonucléotide à double structure tige-boucle objet présentant, à son extrémité 5' ou à son extrémité 3', une structure comprenant une entité E apte à se lier, directement ou indirectement, à au moins un analyte d'intérêt. La présente invention concerne également l'utilisation d'un tel oligonucléotide à double structure tige-boucle pour détecter et éventuellement quantifier au moins un analyte éventuellement présent dans un échantillon liquide, via une amplification isotherme médiée par des boucles à deux amorces.

## Description

### DOMAINE TECHNIQUE

La présente invention concerne le domaine des outils et procédés de détection de molécules d'intérêt.

Plus particulièrement, la présente invention propose un oligonucléotide à double structure tige-boucle particulier, utile notamment pour détecter et éventuellement quantifier au moins un analyte d'intérêt dans un échantillon liquide mettant en œuvre une amplification LAMP (pour « Loop-mediated isothermal AMPlification ») à deux amorces. La présente invention concerne également un tel procédé de détection et d'éventuelle quantification.

### ÉTAT DE LA TECHNIQUE ANTÉRIEURE

De nombreux travaux et études dans des domaines englobant notamment la recherche académique ; la recherche et les analyses, cliniques et diagnostiques, avec des essais réalisés sur des prélèvements de sang ou des biopsies cellulaires ou tissulaires; la surveillance environnementale; la surveillance civile; la sécurité alimentaire et notamment le contrôle qualité ; les analyses criminelles sur traces... impliquent la détection de biomarqueurs, de molécules d'intérêt ou de contaminants dans un échantillon et notamment dans un échantillon liquide.

Plusieurs techniques ont été mises au point à cet effet.

Une première technique immuno-enzymatique de détection intitulée méthode ELISA (pour « Enzyme-Linked ImmunoSorbent Assay ») a été développée au début des années 1970. Dans cette technique, un sandwich entre deux anticorps spécifiques et la cible peut être réalisé. Le premier anticorps est utilisé pour capturer la cible, par exemple, au fond d'un puits, alors que le deuxième anticorps sert à l'amplification enzymatique, directement ou via un anticorps secondaire couplé à une enzyme comme la HRP (pour « Horse Radish Peroxidase »). La méthode ELISA met en œuvre un anticorps et permet une quantification de la cible au moyen d'une gamme de quantification réalisée au préalable ou simultanément. Cette méthode est encore actuellement utilisée et de nombreuses variantes existent. En effet, un aptamère qui est une séquence oligonucléotidique sélectionnée spécifiquement pour son interaction avec un analyte ou toute autre séquence oligonucléotidique ayant une affinité spécifique envers un analyte peut être utilisé à la place d'un anticorps et appliqué comme sonde dans une méthode ELISA. Les aptamères constituent une bonne alternative aux anticorps dans la mesure où ils peuvent détecter de petites molécules et où ils sont très spécifiques d'une cible, beaucoup plus faciles à produire et à manipuler, plus stables et moins coûteux.

Cependant, lorsque la cible à détecter est présente en très faibles concentrations dans l'échantillon, la technique ELISA avec anticorps ou aptamères s'avère inefficace. Pour résoudre ce problème, l'amplification d'une sonde ADN est généralement nécessaire.

C'est pourquoi, d'autres méthodes de détection ont été développées mettant en œuvre une amplification en chaîne par polymérase (ou PCR pour « Polymerase Chain Reaction »). A titre d'exemples particuliers de telles méthodes, on peut citer l'immuno-PCR et l'aptaméro-PCR.

Dans l'immuno-PCR, l'amplification enzymatique de la technique ELISA est remplacée par une amplification d'ADN. Le second anticorps est alors couplé à une séquence ADN qui est exponentiellement amplifiée par PCR. Cette méthode est très spécifique et quantitative mais elle nécessite toujours la présence de deux anticorps spécifiques de la cible et notamment le développement d'un anticorps couplé à un ADN, ce qui rend cette méthode compliquée et coûteuse.

L'aptaméro-PCR, quant à elle, permet de remplacer l'anticorps secondaire par un oligonucléotide contenant une séquence d'aptamère spécifique de la cible à détecter. La détection est obtenue par l'amplification directe de cette sonde par PCR. Cette méthode permet d'atteindre une limite de détection de quelques picomolaires. Cependant, l'immuno-PCR et l'aptaméro-PCR présentent, toutes deux, les inconvénients de la PCR à savoir la nécessité de cycles de températures longs et comportant différentes températures.

Enfin, une autre méthode d'amplification a été développée pour remplacer la PCR dans le cadre de l'amplification de doubles brins d'ADN. Cette méthode appelée LAMP et brevetée **[1]** présente l'avantage majeur d'être isotherme, l'amplification étant réalisée à une température constante typiquement comprise entre 60°C et 65°C.

Ainsi, une méthode de détection de protéines avec amplification LAMP a été décrite par Pourhassan-Moghaddam *et al,* 2013 **[2].** Elle met en œuvre, d'une part, un anticorps ou un aptamère fixé à une surface pour capturer la cible et, d'autre part, un oligonucléotide contenant une séquence aptamère qui reconnaît la cible. Cet oligonucléotide est, par la suite, amplifié par la méthode LAMP classique avec quatre amorces.

Cependant, le design complexe des quatre amorces indispensables à la méthode d'amplification LAMP rend ce procédé complexe et coûteux à mettre en œuvre, malgré le développement de nombreux logiciels de design des amorces LAMP comme, par exemple, LAMP designer (OPtigene) ou primer Explorer.

Enfin, une méthode de détection de micro-ARNs (mi-ARNs) par une méthode LAMP avec deux amorces a été développée **[3,4].** Cette méthode présente l'avantage d'être quantitative. Toutefois, elle ne permet de détecter que de l'ARN à partir duquel est formé un fragment d'ADN complémentaire (ADNc) au moyen d'une réaction de transcription inverse. Deux sondes d'ADN en épingle à cheveux désignées H1 et H2 dans Du *et al,* 2016 **[4]** sont conçues, la sonde H1 présentant à son extrémité 3' une extension complémentaire à la moitié du fragment d'ADNc et la sonde H2 présentant à son extrémité 5' une extension complémentaire à l'autre moitié du fragment d'ADNc. En présence du fragment d'ADNc, les sondes H1 et H2 s'hybrident sur ce dernier et, si aucun mésappariement n'existe entre les extensions des sondes H1 et H2 et le fragment d'ADNc, une liaison covalente entre les deux sondes d'ADN H1 et H2 peut être formée grâce à une ligase haute-fidélité comme la *Taq* DNA ligase. Il est donc clair que cette méthode de détection de mi-ARNs n'est pas générique puisqu'elle nécessite la préparation de deux sondes spécifiques de chaque mi-ARN à détecter. Par ailleurs, cette méthode implique plusieurs étapes avant la mise en œuvre de la technique LAMP avec une étape de transcription inverse et une étape de ligation, cette dernière pouvant être complexe avec un rendement possiblement faible et la nécessité d'utiliser une enzyme supplémentaire. Malgré cela, dans leur publication Du *et al,* 2016 **[4],** les auteurs insistent sur le fait que cette étape est primordiale notamment pour une meilleure discrimination des mutations dans les mi-ARNs (phrase pontant les colonnes de la page 12722).

Les inventeurs ont déjà mis au point une méthode innovante permettant de réaliser une amplification LAMP avec seulement deux amorces **[5].** Cette méthode permet de détecter des analytes d'intérêt de nature variée dans un échantillon et ne présente pas les inconvénients des méthodes de l'état de la technique. Elle met en œuvre une séquence se présentant sous forme d'un oligonucléotide à double structure tige-boucle, servant de matrice à l'amplification LAMP. Elle permet de combiner la reconnaissance d'analytes par des brins oligonucléotidiques et l'amplification LAMP à deux amorces, et donne une détection à haute sensibilité. En effet, l'oligonucléotide à double structure tige-boucle mis en œuvre intègre, au sein de sa structure, une entité de détection de l'analyte d'intérêt. En d'autres termes, l'oligonucléotide présente, au sein de sa structure, au moins une séquence spécifique de l'analyte à détecter, comme, par exemple, un aptamère ou au moins une séquence complémentaire à une partie de la séquence nucléotidique de l'analyte à détecter lorsque ce dernier est une molécule nucléotidique. Dans une telle configuration, seule une séquence nucléotidique peut être utilisée comme entité de détection d'analyte, et celle-ci est intégrée dans la structure à amplifier, rendant son utilisation unique pour chaque application.

Les inventeurs se sont donc fixé pour but de proposer un outil utile dans un procédé permettant de détecter des molécules d'intérêt de nature variée dans un échantillon, ledit procédé ne présentant pas les inconvénients des méthodes de l'état de la technique et d'améliorer encore l'oligonucléotide à double structure tige-boucle décrit dans **[5].**

### EXPOSÉ DE L'INVENTION

La présente invention permet de résoudre des problèmes techniques et inconvénients précédemment listés. En effet, les inventeurs ont proposé un nouvel outil oligonucléotidique notamment utile dans un procédé de détection et éventuellement de quantification d'un analyte, qui permet de profiter des avantages de l'amplification LAMP à deux amorces, à savoir méthode isotherme, quantifiable et ne nécessitant que deux amorces.

L'outil oligonucléotidique objet de la présente invention est un oligonucléotide à double structure tige-boucle. Les expressions et terme « oligonucléotide à double structure tige-boucle », « oligonucléotide à deux structures tige-boucle », « oligonucléotide à structure d'haltère » et « haltère » sont équivalents et utilisables de façon interchangeable dans le cadre de la présente invention.

Les inventeurs ont montré qu'il était possible d'introduire, au niveau de l'une quelconque des extrémités de cet haltère (i.e. extrémité 5' ou 3'), une entité spécifique de l'analyte à détecter et qui permet également une amplification LAMP à deux amorces (voir point I.2. de la partie expérimentale ci-après). Cette amplification est même plus rapide que l'amplification obtenue avec un haltère tel que défini dans **[5]** i.e. un haltère dans l'une quelconque des portions duquel a été introduite une séquence nucléotidique spécifique de l'analyte à détecter. A noter que l'amplification LAMP du procédé de détection objet de **[5]** est plus rapide, moins complexe à réaliser et moins coûteuse qu'une amplification LAMP à 4 amorces, notamment en raison de la réduction du nombre d'amorces. Ces propriétés sont maintenues et voire améliorées en utilisant l'haltère objet de la présente invention.

L'oligonucléotide à double structure tige-boucle objet de la présente invention répond à la formule (I) suivante :

5'-F1c-F2-F0'-F1-Int-B1c-B0'-B2c-B1-3' (I)

dans laquelle
la portion F1c présente une séquence nucléotidique complémentaire à la séquence nucléotidique de la portion F1 moyennant quoi la portion F1c et la portion F1 s'hybrident pour former la tige de la première structure tige-boucle,
F0' séparant la portion F2 et la portion F1 est soit une liaison covalente, soit une portion comprenant au moins un nucléotide,
la portion F2-F0' forme la boucle de la première structure tige-boucle, désignée ci-après boucle F0,
la portion B1 présente une séquence nucléotidique complémentaire à la séquence nucléotidique de la portion B1c moyennant quoi la portion B1 et la portion B1c s'hybrident pour former la tige de la seconde structure tige-boucle,
B0' séparant la portion B1c et la portion B2c est soit une liaison covalente, soit une portion comprenant au moins un nucléotide,
la portion B0'-B2c forme la boucle de la seconde structure tige-boucle, désignée ci-après boucle B0,
Int séparant la portion F1 et la portion B1c est soit une liaison covalente, soit une portion comprenant au moins un nucléotide, et
ledit oligonucléotide présente, à son extrémité 5' et/ou à son extrémité 3', une structure comprenant au moins une entité E apte à se lier, directement ou indirectement, à un analyte d'intérêt.

Plus particulièrement, l'oligonucléotide objet de l'invention présente, à son extrémité 5' ou à son extrémité 3', une structure comprenant au moins une entité E apte à se lier, directement ou indirectement, à un analyte d'intérêt.

L'oligonucléotide à double structure tige-boucle objet de la présente invention se distingue clairement de l'oligonucléotide décrit dans **[3,4].** Dans ce dernier, la séquence apte à reconnaître l'ADNc obtenu à partir du mi-ARN à détecter correspond à la portion Int telle que définie dans la formule (I) ci-dessus. Cette localisation est essentielle au procédé décrit dans **[3,4].** De même, l'oligonucléotide à double structure tige-boucle objet de la présente invention est différent de l'oligonucléotide décrit dans **[5].** Ce dernier présente au moins une séquence nucléotidique reconnaissant, de façon spécifique, l'analyte à détecter dans l'une quelconque des portions de l'oligonucléotide (i.e. F1c, F2, F0', F1, Int, B1c, B0', B2c et B1) ou dans plusieurs de ces portions. Il est indiqué dans **[5]** que, dans l'oligonucléotide à double structure tige-boucle mis en œuvre, le nucléotide à l'extrémité 5' de la portion F1c est lié, au moyen de liaisons hydrogène, avec le nucléotide correspondant dans la portion F1 et le nucléotide à l'extrémité 3' de la portion B1 est lié, au moyen de liaisons hydrogène, avec le nucléotide correspondant dans la portion B1c. Un tel enseignement aurait dissuadé l'homme du métier de lier, à l'extrémité 5' de la portion F1c ou à l'extrémité 3' de la portion B1, une structure telle que définie dans la présente invention.

En d'autres termes, l'oligonucléotide à double structure tige-boucle objet de la présente invention répond à l'une quelconque des formules (II), (III) et (IV) suivantes :

S-F1c-F2-F0'-F1-Int-B1c-B0'-B2c-B1 (II)

F1c-F2-F0'-F1-Int-B1c-B0'-B2c-B1-S (III)

et

S-F1c-F2-F0'-F1-Int-B1c-B0'-B2c-B1-S' (IV)

dans lesquelles
F0', Int et B0' et les portions F1c, F2, F1, B1c, B2c et B1 sont tels que précédemment définis, et
S et S', identiques ou différents, représentent une structure comprenant au moins une entité E apte à se lier, directement ou indirectement, à un analyte d'intérêt.

Plus particulièrement, l'oligonucléotide objet de l'invention l'oligonucléotide à double structure tige-boucle objet de la présente invention répond à la formule (II) ou (III) suivante :

S-F1c-F2-F0'-F1-Int-B1c-B0'-B2c-B1 (II)

F1c-F2-F0'-F1-Int-B1c-B0'-B2c-B1-S (III)

dans lesquelles
F0', Int et B0' et les portions F1c, F2, F1, B1c, B2c et B1 sont tels que précédemment définis, et
S représente une structure comprenant au moins une entité E apte à se lier, directement ou indirectement, à un analyte d'intérêt.

Il est évident que, aux extrémités de l'oligonucléotide de formule (II), on trouve, d'un côté, à la structure S et, de l'autre, l'extrémité 3' de la portion B1. De même, pour l'oligonucléotide de formule (III), ses extrémités correspondent à l'extrémité 5' de la portion F1c et à la structure S.

Dans le cadre de la présente invention, deux séquences nucléotidiques sont complémentaires l'une de l'autre, lorsqu'un nombre suffisant de nucléotides de la première séquence nucléotidique peut se lier, au moyen de liaisons hydrogène, avec les nucléotides correspondants de la deuxième séquence nucléotidique de telle sorte que l'appariement entre les deux séquences nucléotidiques peut se produire. La « complémentarité », telle qu'utilisée ici, se réfère à la capacité d'appariement entre les nucléotides d'une première séquence nucléotidique et d'une seconde séquence nucléotidique. Des nucléotides non complémentaires entre deux séquences nucléotidiques peuvent être tolérés à condition que les deux séquences nucléotidiques restent capables de s'hybrider spécifiquement l'une à l'autre. De plus, une première séquence nucléotidique peut s'hybrider sur un ou plusieurs segments d'une seconde séquence nucléotidique de telle sorte que les segments intermédiaires ou adjacents ne sont pas impliqués dans l'hybridation. Dans certaines formes de mise en œuvre de l'invention, une première séquence nucléotidique ou une partie spécifique de celle-ci, est au moins 70%, 80%, 85%, 86%, 87%, 88%, 89%, 90%, 91%, 92%, 93%, 94%, 95%, 96%, 97%, 98%, 99% ou 100% complémentaire à une deuxième séquence nucléotidique ou une partie spécifique de celle-ci.

Typiquement, dans l'oligonucléotide à double structure tige-boucle mis en œuvre dans l'invention, la portion F1 et la portion F1c comprennent, chacune, de 10 à 35 nucléotides, notamment entre 15 et 25 nucléotides et, à titre d'exemples particuliers, 19, 20, 21 ou 22 nucléotides. La température de fusion (Tm) de F1-F1c est notamment supérieure à la température utilisée lors de l'amplification et, en particulier, supérieure de 5°C à 7°C. L'homme du métier pourra trouver des informations additionnelles quant à ces portions dans [1].

Typiquement, la portion B1 et la portion B1c comprennent, chacune, de 10 à 35 nucléotides, notamment entre 15 et 30 nucléotides et, à titre d'exemples particuliers, 18 ou 25 nucléotides. La température Tm de B1-B1c est notamment supérieure à la température utilisée lors de l'amplification et, en particulier, supérieure de 5°C à 7°C. L'homme du métier pourra trouver des informations additionnelles quant à ces portions dans **[1].**

En particulier, la portion F2 comprend de 8 à 30 nucléotides et, à titre d'exemple particulier, 21 ou 24 nucléotides. Dans l'oligonucléotide selon la présente invention, F0' est soit une liaison covalente reliant la portion F2 et la portion F1 moyennant quoi la boucle F0 est formée par la portion F2, soit une portion comprenant au moins un nucléotide. Dans ce dernier cas, la portion F0' est telle que la boucle F0 formée par la portion F2-F0' comprend de 10 à 70 nucléotides.

De plus, la portion B2c comprend de 8 à 35 nucléotides, notamment entre 15 et 30 nucléotides et, à titre d'exemples particuliers, 18 ou 22 nucléotides. Dans l'oligonucléotide selon la présente invention, B0' est soit une liaison covalente reliant la portion B1c et la portion B2c moyennant quoi la boucle B0 est formée par la portion B2c, soit une portion comprenant au moins un nucléotide. Dans ce dernier cas, la portion B0' est telle que la boucle B0 formée par la portion B0'-B2c comprend de 10 à 70 nucléotides.

Dans l'oligonucléotide selon la présente invention, lorsque Int représente une portion comprenant au moins un nucléotide, cette dernière peut comprendre jusqu'à 70 nucléotides.

Avantageusement, l'oligonucléotide à double structure tige-boucle selon la présente invention comprend moins de 200 nucléotides, notamment moins de 180 nucléotides, en particulier moins de 170 nucléotides, plus particulièrement moins de 160 nucléotides et tout particulièrement moins de 150 nucléotides. Dans certains modes de réalisation, l'oligonucléotide à double structure tige-boucle selon la présente invention peut comprendre moins de 140 nucléotides, notamment moins de 130 nucléotides, en particulier moins de 120 nucléotides, plus particulièrement moins de 110 nucléotides et tout particulièrement moins de 100 nucléotides, notamment du fait que les portions Int, F0' et B0' sont optionnelles et que la structure S peut ne comprendre aucun nucléotide. Avec une telle taille, la synthèse chimique de cet oligonucléotide est facilitée et le coût de cette synthèse et, par conséquent, le coût du procédé selon l'invention sont moindres.

L'oligonucléotide à double structure tige-boucle selon l'invention présente, à l'extrémité 5' de la portion F1c (cas selon la formule (II) ou (IV)) ou à l'extrémité 3' de la portion B1 (cas selon la formule (III) ou (IV)), une structure S ou S' comprenant au moins une entité E apte à se lier, directement ou indirectement, à un analyte d'intérêt.

Par conséquent, l'oligonucléotide selon l'invention présente :
- une portion F1 et une portion F1c comprenant, chacune, de 10 à 35 nucléotides, et/ou
- une portion B1 et une portion B1c comprenant, chacune, de 10 à 35 nucléotides, et/ou
- une portion F2 comprenant de 8 à 30 nucléotides et/ou
- une portion B2c comprenant de 8 à 35 nucléotides.

La structure S ou S' dans l'oligonucléotide selon l'invention peut ne comprendre qu'une entité E telle que précédemment définie. Dans cette première variante, la structure S consiste en l'entité E. En d'autres termes, l'entité E est liée, via une liaison covalente, à l'extrémité 5' de la portion F1c (cas selon la formule (II) ou (IV)) ou à l'extrémité 3' de la portion B1 (cas selon la formule (III) ou (IV)).

Dans une deuxième variante, la structure S ou S' dans l'oligonucléotide selon l'invention peut comprendre, en plus d'une entité E telle que précédemment définie, au moins un autre élément permettant de lier l'entité E à l'extrémité 5' de la portion F1c (cas selon la formule (II) ou (IV)) ou à l'extrémité 3' de la portion B1 (cas selon la formule (III) ou (IV)). Cet élément additionnel est connu sous l'expression « bras de liaison » et sert à améliorer l'accessibilité de l'entité E. L'homme du métier connaît différents exemples de tels éléments utilisables dans le cadre de la présente invention. A titre d'exemples, on peut citer une molécule nucléotidique telle que précédemment définie et notamment une séquence comprenant plusieurs bases thymine et notamment 10 bases thymine, un polymère, comme, par exemple un poly-éthylène glycol (PEG) ou une structure moléculaire streptavidine-biotine. Les liaisons mises en œuvre dans cette variante sont des liaisons non covalentes et de faible énergie telles que des liaisons hydrogène ou des liaisons de Van der Waals et/ou des liaisons de forte énergie de type liaisons covalentes.

Dans une troisième variante, la structure S ou S' dans l'oligonucléotide selon l'invention peut comprendre plusieurs entités E telles que précédemment définies, identiques ou différentes. Dans cette variante, deux entités E successives peuvent éventuellement être séparées par un bras de liaison. De même, la première de ces entités peut éventuellement être liée à l'extrémité 5' de la portion F1c (cas selon la formule (II) ou (IV)) ou à l'extrémité 3' de la portion B1 (cas selon la formule (III) ou (IV)) via un bras de liaison. Enfin, lorsque la structure S ou S' présente plusieurs bras de liaison, ces derniers peuvent être identiques ou différents.

Dans un premier mode de réalisation, la ou les entités E présentes au niveau de la structure S ou S' dans l'oligonucléotide selon la présente invention sont aptes à se lier directement à au moins un analyte d'intérêt. Une entité E selon ce mode de réalisation peut également être désignée, dans la présente, par l'expression « entité de reconnaissance ».

Cette entité peut être toute molécule capable de former avec l'analyte à détecter une paire de liaison, l'entité E et l'analyte correspondant aux deux partenaires de cette paire de liaison. Les liaisons mises en œuvre dans la liaison analyte-entité sont avantageusement des liaisons non covalentes et de faible énergie telles que des liaisons hydrogène ou des liaisons de Van der Waals et/ou des liaisons de forte énergie de type liaisons covalentes. Il convient de noter qu'avec les oligonucléotides décrits dans [5], une liaison de forte énergie entre l'analyte et la séquence nucléotidique le reconnaissant, de façon spécifique et présente au sein de l'oligonucléotide n'était pas possible et ce, pour éviter d'affecter l'amplification LAMP.

La ou les entités de reconnaissance utilisées sont donc dépendantes du ou des analytes à détecter. En fonction de ce ou des derniers, l'homme du métier saura, sans effort inventif, choisir la ou les entités les plus adaptées. Une entité de reconnaissance peut être choisie, par exemple, dans le groupe constitué par un glucide ; un peptide tel qu'un peptide antimicrobien ou un MIP pour « MHC class I peptide » i.e. un peptide associé au CMH-1 (pour « Complexe Majeur d'Histocompatibilité-1 »); un antigène ; un épitope ; une protéine ; une glycoprotéine ; une enzyme ; un substrat enzymatique; un récepteur membranaire ou nucléaire; un agoniste ou un antagoniste d'un récepteur membranaire ou nucléaire ; une toxine ; un anticorps polyclonal ou monoclonal ; un fragment d'anticorps tel qu'un fragment Fab, F(ab')₂, Fv ou un domaine hypervariable (ou CDR pour « Complementarity Determining Region »); une molécule nucléotidique ; et un aptamère.

De fait, l'oligonucléotide selon la présente invention se distingue encore des autres oligonucléotides à double structure tige-boucle de l'art antérieur, utiles dans la reconnaissance d'analytes d'intérêt. Dans ces derniers, une entité formant une paire de liaison avec un analyte d'intérêt se présente obligatoirement sous forme d'une séquence nucléotidique, alors qu'elle peut être de nature glucidique, peptidique, lipidique et/ou nucléotidique dans le cadre de la présente invention.

L'expression « molécule nucléotidique » utilisée dans la présente est équivalente aux termes et expressions suivants : « acide nucléique », « polynucléotide », « séquence nucléotidique », « séquence polynucléotidique » et « séquence oligonucléotidique ». Par « molécule nucléotidique », on entend, dans le cadre de la présente invention, un chromosome; un gène; un polynucléotide régulateur; un ADN, simple-brin ou double-brin, génomique, chromosomique, chloroplastique, plasmidique, mitochondrial, recombinant ou complémentaire; un ARN total; un ARN messager; un ARN ribosomal (ou ribozyme) ; un ARN de transfert ; un micro-ARN ; une séquence faisant office d'aptamère ; un acide nucléique peptidique ; un acide nucléique verrouillé (ou LNA pour « Locked Nucleic Acid »); un morpholino ; une portion ou un fragment de ceux-ci.

Dans un second mode de réalisation notamment illustré au point III de la partie expérimentale ci-après, la ou les entités E présentes au niveau de la structure S ou S' dans l'oligonucléotide selon la présente invention sont aptes à se lier indirectement à un ou des analytes d'intérêt. En d'autres termes, cette ou ces entités E sont indépendantes du ou des analytes d'intérêt à détecter et cette détection nécessite l'utilisation d'un intermédiaire de liaison apte à se lier, d'une part, à l'oligonucléotide selon ce second mode de réalisation de l'invention et, d'autre part, à ou aux analytes d'intérêt. Ce partenaire de liaison correspond à une molécule comprenant une première portion P1 apte à se lier à une entité E présente dans la structure S ou S' de l'oligonucléotide et une seconde portion P2 apte à se lier à au moins un analyte d'intérêt. La portion P2 de cette molécule correspond à une entité de reconnaissance telle que définie précédemment ou comprend plusieurs entités de reconnaissance telles que définies précédemment, identiques ou différentes. Ainsi, tout ce qui a été décrit ci-dessus pour l'entité ou les entités de reconnaissance s'applique *mutatis mutandis* à la portion P2. Dans ce partenaire de liaison, la portion P1 et la portion P2 sont couplées l'une à l'autre au moyen d'une liaison covalente. En variante, la portion P1 et la portion P2 peuvent être couplées l'une à l'autre au moyen d'un bras de liaison tel que précédemment défini.

Une entité E de ce second mode de réalisation peut être toute molécule capable de former une paire de liaison avec la portion P1 du partenaire de liaison, l'entité E et la portion P1 correspondant aux deux partenaires de cette paire de liaison. Les liaisons mises en œuvre dans la liaison entité-portion P1 sont des liaisons non covalentes et de faible énergie telles que des liaisons hydrogène ou des liaisons de Van der Waals.

La présente invention concerne un complexe non-covalent, formé par un oligonucléotide selon le second mode de réalisation et une molécule comprenant une première portion P1 apte à se lier à ladite au moins une entité E de cet oligonucléotide et une seconde portion P2 apte à se lier à au moins un analyte d'intérêt. En d'autres termes, ce complexe non-covalent est formé par un oligonucléotide à double structure tige-boucle dont la structure S ou S' comprend au moins une entité E apte à se lier indirectement à au moins un analyte d'intérêt tel que précédemment défini et une molécule comprenant une première portion P1 apte à se lier à cette au moins une entité E présente dans la structure S ou S' et une seconde portion P2 apte à se lier à au moins un analyte d'intérêt. Un tel complexe peut être désigné par l'expression « complexe de liaison ».

La présente invention concerne également l'utilisation d'un oligonucléotide à double structure tige-boucle tel que précédemment défini ou un complexe de liaison tel que précédemment défini pour détecter et éventuellement quantifier au moins un analyte éventuellement présent dans un échantillon liquide.

Dans un mode de réalisation particulier, la présente invention concerne un procédé pour détecter et éventuellement quantifier au moins un analyte éventuellement présent dans un échantillon liquide, comprenant les étapes suivantes :
i) mettre en contact ledit échantillon liquide avec la surface d'un support solide comprenant au moins une zone active sur laquelle au moins une sonde apte à lier ledit au moins un analyte est immobilisée ;
ii) mettre en contact ladite surface avec une solution contenant soit au moins un oligonucléotide à double structure tige-boucle dont la structure comprend une entité E apte à se lier directement à au moins un analyte d'intérêt tel que précédemment défini, soit un complexe de liaison tel que précédemment défini, ledit oligonucléotide et ledit complexe ayant été préparés préalablement à ladite mise en contact ;
iii) éliminer l'excès d'oligonucléotides ou l'excès de complexes de liaison n'ayant pas réagi lors de la mise en contact de l'étape ii) ;
iv) mettre en contact ladite surface avec deux amorces d'amplification isotherme médiée par des boucles dans des conditions permettant l'amplification dudit oligonucléotide ;
v) détecter et éventuellement quantifier le produit de l'amplification dudit oligonucléotide (moyennant quoi ledit au moins un analyte est détecté et éventuellement quantifié).

Dans un autre mode de réalisation particulier, la présente invention concerne un procédé pour détecter et éventuellement quantifier au moins un analyte éventuellement présent dans un échantillon liquide, comprenant les étapes suivantes :
i') mettre en contact ledit échantillon liquide avec la surface d'un support solide comprenant au moins une zone active sur laquelle au moins une sonde apte à lier ledit au moins un analyte est immobilisée ;
ii₁') mettre en contact ladite surface avec une solution contenant au moins une molécule comprenant une première portion P1 apte à se lier à l'entité E présente dans la structure de l'oligonucléotide selon le second mode de réalisation tel que précédemment défini et une seconde portion P2 apte à se lier audit au moins un analyte d'intérêt ;
ii₂') éliminer l'excès de molécules n'ayant pas réagi lors de la mise en contact de l'étape iii') ;
ii₃') mettre en contact ladite surface avec une solution contenant au moins un oligonucléotide à double structure tige-boucle dont la structure comprend une entité E apte à se lier indirectement audit analyte d'intérêt tel que précédemment défini, ledit oligonucléotide ayant été synthétisé préalablement à ladite mise en contact ;
iii') éliminer l'excès d'oligonucléotides n'ayant pas réagi lors de la mise en contact de l'étape ii₃') ;
iv') mettre en contact ladite surface avec deux amorces d'amplification isotherme médiée par des boucles dans des conditions permettant l'amplification dudit oligonucléotide ;
v') détecter et éventuellement quantifier le produit de l'amplification dudit oligonucléotide (moyennant quoi ledit au moins un analyte est détecté et éventuellement quantifié).

L'échantillon liquide mis en œuvre dans le cadre de la présente invention est un liquide susceptible de contenir un ou des analytes à détecter et éventuellement à quantifier. Il peut être de nature et d'origine très variées.

Cet échantillon liquide est avantageusement choisi dans le groupe constitué par un fluide biologique ; un fluide végétal tel que sève, nectar et exsudat racinaire; un prélèvement dans un milieu de culture ou dans un réacteur de culture biologique comme une culture cellulaire d'eucaryotes supérieurs, de levures, de champignons, d'algues ou de bactéries; un liquide obtenu à partir d'une ou plusieurs cellules animales ou végétales ; un liquide obtenu à partir d'un tissu animal ou végétal ; un prélèvement dans une matrice alimentaire; un prélèvement dans un réacteur chimique ; de l'eau de ville, de rivière, de mer, de piscines, de tours aéro-réfrigérées ou d'origine souterraine; un prélèvement à partir d'un effluent industriel liquide; de l'eau usée provenant notamment d'élevages intensifs ou d'industries du domaine chimique, pharmaceutique ou cosmétique ; un prélèvement provenant d'une filtration d'air ou d'un revêtement; un prélèvement sur un objet tel qu'un fragment de tissu, un habit, une semelle, une chaussure, un outil ou une arme; un produit pharmaceutique ; un produit cosmétique ; un parfum ; un échantillon de terre ou un de leurs mélanges.

Dans le cadre de la présente invention, on entend par « prélèvement » tout type de collecte d'échantillons, par exemple, par contact, raclage, forage, découpage, poinçonnage, broyage, lavage, rinçage, aspiration ou pompage.

Le fluide biologique est avantageusement choisi dans le groupe constitué par le sang tel que du sang entier ou du sang entier anti-coagulé, le sérum sanguin, le plasma sanguin, la lymphe, la salive, le crachat, les larmes, la sueur, le sperme, l'urine, les selles, le lait, le liquide céphalo-rachidien, le liquide interstitiel, un fluide isolé de moelle osseuse, un mucus ou fluide du tractus respiratoire, intestinal ou génito-urinaire, des extraits cellulaires, des extraits de tissus et des extraits d'organes. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement ou un lavage. Les étapes de récupération et d'isolement de ces différents fluides à partir du corps humain ou animal sont réalisées préalablement à la mise en œuvre du procédé selon l'invention.

De même, si un des prélèvements envisagés ne permet pas de mettre en œuvre le procédé de l'invention, par exemple du fait de sa nature gazeuse ou solide, de sa concentration ou des éléments qu'il contient tels que résidus solides, déchets, suspension ou molécules interférentes, le procédé de l'invention comprend en outre une étape préalable de préparation de l'échantillon liquide avec éventuellement une mise en solution du prélèvement par les techniques connues de l'homme du métier telles que filtration, précipitation, dilution, distillation, mélange, concentration, lyse, etc.

Par ailleurs, il est possible d'ajouter, à l'échantillon liquide, des quantités connues d'au moins un analyte à détecter. On parle alors d'échantillon liquide dopé par au moins un analyte, ce dopage permettant d'avoir un contrôle positif notamment lorsque l'échantillon liquide est complexe et éventuellement d'obtenir une quantification du ou des analytes à détecter initialement contenus dans l'échantillon liquide.

Un analyte à détecter et éventuellement à quantifier dans l'échantillon liquide peut être choisi dans le groupe constitué par une molécule d'intérêt environnemental tel qu'un pesticide ; une molécule d'intérêt biologique ; une molécule d'intérêt pharmacologique ; une toxine ; un glucide tel que du glucose ; un lipide tel que du cholestérol ; un peptide ; un antigène ; un épitope ; une protéine ; une glycoprotéine ; une enzyme ; un substrat enzymatique ; un récepteur nucléaire ou membranaire ; un agoniste ou un antagoniste d'un récepteur nucléaire ou membranaire ; une hormone ; un anticorps polyclonal ou monoclonal ; un fragment d'anticorps tel qu'un fragment Fab, F(ab')₂, Fv ou un domaine hypervariable ; une molécule nucléotidique telle que précédemment définie; des ions tels que des ions mercure ou des ions plomb; une cellule eucaryote ; une cellule procaryote et un virus.

A noter que, dans certaines formes de mise en œuvre, un analyte à détecter et éventuellement à quantifier dans l'échantillon liquide peut être défini comme une petite molécule i.e. une molécule dont le poids moléculaire est inférieur ou égal à 10000 daltons et notamment inférieur ou égal à 8000 daltons. Cette petite molécule peut appartenir à l'un quelconque des éléments des listes d'analytes ci-dessus.

L'étape i) ou i') du procédé selon la présente invention met en œuvre une sonde apte à lier ledit au moins un analyte immobilisée sur la surface d'un support solide. La sonde utilisée pour fonctionnaliser une zone active de la surface du support solide est toute molécule capable de former avec un analyte à détecter une paire de liaison, la sonde et l'analyte correspondant aux deux partenaires de cette paire de liaison. Les liaisons mises en œuvre dans la liaison analyte-sonde sont avantageusement soit des liaisons non covalentes et de faible énergie telles que des liaisons hydrogène ou des liaisons de Van der Waals, soit des liaisons de forte énergie de type liaisons covalentes.

La sonde utilisée est donc dépendante d'un analyte à détecter. En fonction de cet analyte, l'homme du métier saura, sans effort inventif, choisir la sonde la plus adaptée. Elle peut être choisie, par exemple, dans le groupe constitué par un glucide ; un peptide tel qu'un peptide antimicrobien ou un MIP i.e. un peptide associé au CMH-1 (pour « Complexe Majeur d'Histocompatibilté-1 »); un antigène ; un épitope ; une protéine ; une glycoprotéine ; une enzyme ; un substrat enzymatique ; un récepteur membranaire ou nucléaire; un agoniste ou un antagoniste d'un récepteur membranaire ou nucléaire ; une toxine ; un anticorps polyclonal ou monoclonal ; un fragment d'anticorps tel qu'un fragment Fab, F(ab')₂, Fv ou un domaine hypervariable ; une molécule nucléotidique telle que précédemment définie ; et un aptamère.

Il est évident que, lors du choix de la sonde la mieux adaptée, il convient de tenir compte de l'entité de reconnaissance mise en œuvre ultérieurement. La sonde et l'entité de reconnaissance peuvent reconnaître des zones ou éléments distincts au niveau de l'analyte à détecter. En variante, la sonde et l'entité de reconnaissance peuvent cibler le même élément au niveau de l'analyte à détecter. Cette variante correspond notamment au cas où l'analyte à détecter est une bactérie ou un virus et l'élément à cibler un élément de surface présent au niveau de plusieurs zones de la surface bactérienne ou virale.

Tout support solide permettant de mettre en œuvre la présente invention est utilisable. Il peut s'agir, par exemple, d'un support de biopuce tel que ceux classiquement utilisés en silicium, en verre, en métal, en polymère ou en plastique. Ce support solide peut également consister en des particules et notamment des particules de silice, des particules polymériques et/ou des particules magnétisables.

La fonctionnalisation de la zone active de la surface du support solide par une sonde telle que précédemment définie peut être effectuée par toute technique adaptée permettant la fixation d'un composé sur un support solide. On peut en particulier envisager une simple adsorption, des liaisons ioniques, des liaisons hydrogène, des interactions électrostatiques, des interactions hydrophobes, des liaisons de Van der Waals ou un greffage covalent.

Dans une forme de mise en œuvre particulière de la présente invention, la surface du support solide présente des groupements fonctionnels grâce auxquels la ou les sondes sont capables de s'immobiliser. De façon avantageuse, ces groupements fonctionnels sont choisis parmi les groupements carboxyliques, des entités radicalaires, les fonctions alcools, amines, amides, époxy ou thiols. Ces groupements sont portés, de façon intrinsèque ou par fonctionnalisation, par la zone active de la surface du support solide. La ou les sondes peuvent également présenter de tels groupements fonctionnels, de façon intrinsèque ou par fonctionnalisation.

Dans une première variante de la présente invention, la ou les sondes peuvent être immobilisées de façon directe au niveau de la surface fonctionnalisée ou non. Un support solide « coaté » avec une protéine telle que la streptavidine est un exemple d'immobilisation directe, dans lequel la sonde doit être fonctionnalisée par une biotine.

Dans une seconde variante de la présente invention, la ou les sondes peuvent être immobilisées de façon indirecte au niveau de la surface fonctionnalisée ou non. Cette immobilisation indirecte fait intervenir un bras espaceur (ou agent de jonction) lié, d'une part, à la surface et, d'autre part, à une sonde. Un tel bras espaceur est notamment utilisé pour améliorer l'accessibilité à la sonde. A titre d'exemples illustratifs, on peut citer les réactifs silanes mis en œuvre pour le greffage de sondes sur du verre, la complexation de produits thiolés sur des surfaces d'or et l'immobilisation de sondes dans des matrices polymères. De plus, lorsque la ou les sondes sont une ou des molécules nucléotidiques telles que précédemment définies, un tel bras espaceur peut se présenter sous forme d'une séquence comprenant plusieurs bases thymine et notamment 10 bases thymine.

Les liaisons mises en œuvre au cours d'une immobilisation directe ou indirecte peuvent être toutes liaisons connues de l'homme du métier et notamment des liaisons covalentes, des liaisons ioniques, des liaisons hydrogène, des interactions électrostatiques, des interactions hydrophobes, des liaisons de Van der Waals ou une adsorption.

Avantageusement, une étape de blocage de la surface peut être mise en œuvre préalablement à la fixation de la sonde et/ou suite à cette fixation. La solution de blocage mise en œuvre lors de cette étape de blocage peut comporter un ou plusieurs des composants suivants : albumine comme de la BSA (pour « Bovine Serum Albumin »), ADN génomique comme de l'ADN simple brin et notamment de l'ADN simple brin de sperme de saumon, gélatine, caséine, protéines de lait, sérum, polyéthylène glycol et polymères du type polyvinylpyrrolidone. Un exemple particulier de solution de blocage est une solution contenant de 1% à 5% de BSA et éventuellement 20 µg/mL d'ADN simple brin de sperme de saumon.

L'étape i) ou i') du procédé selon l'invention consiste à mettre en contact l'échantillon liquide tel que précédemment défini avec la surface d'un support solide fonctionnalisée par une ou plusieurs sondes aptes à lier l'analyte à détecter moyennant quoi, si l'échantillon liquide contient cet analyte, ce dernier est capturé au niveau de la ou des sondes.

Typiquement, l'étape i) ou i') de mise en contact peut durer entre 1 min et 24 h, entre 2 min et 12 h, entre 5 min et 6 h, entre 10 min et 3 h, entre 15 min et 2 h, entre 20 min et 1 h et, notamment, de l'ordre de 30 min (i.e. 30 min ± 5 min). Par ailleurs, l'étape i) de mise en contact peut être réalisée à une température comprise entre 4°C et 70°C, notamment entre 10°C et 60°C, en particulier entre 15°C et 45°C et, plus particulièrement, à température ambiante (i.e. 23°C ± 5°C) ou à température physiologique (i.e. 37°C ± 5°C).

Suite à l'étape i) ou i') et préalablement à l'étape ii) ou ii₁') du procédé selon la présente invention, il est possible d'éliminer les éléments présents dans l'échantillon liquide et qui n'ont pas été capturés par la ou les sondes. Toute technique permettant une telle élimination est utilisable dans le cadre de la présente invention. A titre d'exemple, on peut citer le lavage de la surface du support solide ou la séparation de l'échantillon liquide et du support solide comme une séparation physique ou magnétique. Toutefois, cette étape d'élimination est optionnelle puisque ces éléments pourront être éliminés par la suite et notamment lors de l'une quelconque des étapes iii), ii₂') ou iii').

Lorsque cette étape d'élimination est mise en œuvre, la surface du support solide peut être soumise à au moins un rinçage de façon à éliminer toute trace de l'échantillon liquide. Ce rinçage est typiquement effectué avec une solution aqueuse de rinçage conservant l'interaction sonde/analyte ou liant irréversiblement l'analyte et la sonde. Cette solution peut également permettre d'éliminer les interactions non spécifiques. Elle peut comporter un ou plusieurs des composants suivants : un tampon comme un tampon Tris, phosphate, acétate ou borate ; des sels comme KCl, NaCl, (NH₄)₂SO₄, MgCl₂ ou CaCl₂ ; des détergents ou agents tensioactifs comme Tween^{®}, Triton^{®} ou dodécylsulfate de sodium (ou SDS); des agents dénaturants comme formamide ou diméthylsulfoxyde ; un solvant organique comme éthanol, méthanol ou acétonitrile ; et des agents pontants comme formaldéhyde ou glutaraldéhyde. Ce rinçage peut être répété deux fois, trois fois, cinq fois, 10 fois et voire 50 fois en utilisant, à chaque rinçage, une solution de rinçage identique ou différente. Typiquement, le rinçage est répété trois fois avec une solution comprenant (i) du tampon phosphate salin (ou PBS) et 0,1% de Tween^{®} comme du Tween^{®}20, (ii) du PBS, 0,3% de Tween^{®} comme du Tween^{®}20 et 1 M de NaCl ou (iii) une solution saline contenant 0,02 M de PBS, 1,074 M de NaCl et 0,3% de Tween^{®} comme du Tween^{®}20.

De plus, lorsque cette étape optionnelle d'élimination est mise en œuvre, elle est réalisée à une température comprise entre 4°C et 100°C, notamment entre 10°C et 60°C, en particulier entre 15°C et 45°C et, plus particulièrement, à température ambiante ou à température physiologique.

L'étape ii) du procédé selon la présente invention consiste à mettre en contact la surface du support solide sur laquelle un analyte à détecter est éventuellement retenu via la ou les sondes fonctionnalisant cette surface avec une solution contenant au moins un oligonucléotide à double structure tige-boucle, capable de se lier audit analyte ou au moins un complexe de liaison tel que précédemment défini, moyennant quoi, si cet analyte est présent à la surface du support solide, l'oligonucléotide ou le complexe de liaison se lie à ce dernier respectivement via son entité de reconnaissance ou sa portion P2. En d'autres termes, l'oligonucléotide à double structure tige-boucle ou le complexe de liaison est capable de former avec l'analyte à détecter une paire de liaison, l'oligonucléotide ou le complexe de liaison et l'analyte correspondant aux deux partenaires de cette paire de liaison. Les liaisons mises en œuvre dans la liaison oligonucléotide-analyte ou dans la liaison complexe-analyte sont avantageusement des liaisons non covalentes et de faible énergie telles que des liaisons hydrogène ou des liaisons de Van der Waals et/ou des liaisons de forte énergie de type liaisons covalentes.

L'oligonucléotide a été synthétisé préalablement à la mise en contact de l'étape ii) ou ii₃'). En d'autres termes, cette synthèse n'est effectuée ni en présence de la surface du support solide fonctionnalisée par la ou les sondes telles que précédemment définies, ni en présence de l'analyte à détecter. Typiquement, la synthèse de cet oligonucléotide à double structure tige-boucle ne fait pas intervenir deux des amorces habituellement mises en œuvre dans une amplification LAMP à quatre amorces. Avantageusement, cette synthèse est une synthèse chimique classiquement utilisée pour préparer des oligonucléotides.

Lors de l'étape ii) du procédé selon la présente invention, la surface du support solide sur laquelle est éventuellement maintenu au moins un analyte au moyen d'une sonde est mise en contact avec une solution contenant au moins un oligonucléotide à double structure tige-boucle tel que précédemment défini ou un complexe de liaison tel que précédemment défini. Cette solution est avantageusement une solution aqueuse permettant l'interaction haltère ou complexe/analyte. Elle peut comporter un ou plusieurs des composants suivants : un tampon comme un tampon Tris, phosphate, acétate ou borate ; des sels comme KCl, NaCl, (NH₄)₂SO₄, MgCl₂ ou CaCl₂ ; des détergents ou agents tensioactifs comme Tween^{®}, Triton^{®}ou dodécylsulfate de sodium (ou SDS) ; des agents dénaturants comme formamide ou diméthylsulfoxyde ; un solvant organique comme éthanol, méthanol ou acétonitrile; et des agents pontants comme formaldéhyde ou glutaraldéhyde. Avantageusement, lorsque l'oligonucléotide comprend une séquence aptamère pour la reconnaissance de l'analyte, la solution utilisée sera composée des éléments en totalité ou en partie constituant le tampon de sélection de l'aptamère. La solution utilisée lors de l'étape ii) peut, en outre, comprendre un ou plusieurs éléments choisis parmi un ADN génomique comme de l'ADN simple brin et notamment de l'ADN simple brin de sperme de saumon, du sérum, de l'albumine, un polymère de synthèse, un agent bloquant comme l'agent bloquant Denhardt et tout autre élément permettant de limiter l'adsorption non spécifique. Un exemple particulier de solution mise en œuvre lors de l'étape ii) est une solution saline contenant 0,02 M de PBS, 1,074 M de NaCl, 0,3% de détergent Tween20, 20 µg/mL d'ADN de sperme de saumon ainsi que 4% d'agent bloquant Denhardt.

Par ailleurs, l'oligonucléotide à double structure tige-boucle ou le complexe de liaison est présent dans cette solution en une quantité comprise entre 1 aM et 1 mM, et notamment entre 100 aM et 1µM. A titre d'exemples particuliers de concentrations utilisables, on peut citer une concentration de 10 pM, de 100 pM, de 1 nM, ou de 10 nM. Avantageusement, l'oligonucléotide à double structure tige-boucle ou le complexe de liaison est présent dans la solution mise en œuvre lors de l'étape ii) en une concentration de 100 pM.

Typiquement, l'étape ii) de mise en contact peut durer entre 1 min et 24 h, notamment entre 2 min et 12 h et, en particulier, entre 5 min et 6 h. Dans une forme de mise en œuvre particulière, cette mise en contact peut durer entre 10 min et 3 h, entre 15 min et 2 h, entre 20 min et 1 h et, notamment, de l'ordre de 30 min (i.e. 30 min ± 5 min). Dans une autre forme de mise en œuvre, cette mise en œuvre peut durer entre 30 min et 6 h, entre 1 h et 5 h, entre 2 h et 4 h et, notamment, de l'ordre de 3 h (i.e. 3 h ± 15 min). De même, l'étape (ii) de mise en contact peut être réalisée à une température comprise entre 4°C et 100°C, notamment entre 10°C et 60°C, en particulier entre 15°C et 30°C et, plus particulièrement, à température ambiante (i.e. 23°C ± 5°C) ou à température physiologique (i.e. 37°C± 5°C). En effet, une étape préliminaire à faire avant la mise en contact avec la surface pour obtenir un repliement optimal de l'haltère.

Préalablement à cette mise en contact, il est possible de chauffer l'oligonucléotide selon l'invention à une température supérieure à 80°C et notamment de l'ordre de 90°C (i.e. 90°C ± 5°C) pendant 5 min puis de le refroidir jusqu'à la température ambiante en environ 35 min (35 min ± 10 min). Cette étape préalable permet, notamment lorsque l'entité de reconnaissance est un aptamère, de donner à ce dernier une conformation optimale pour la détection de l'analyte.

Tout ce qui a été décrit ci-dessus pour l'étape ii) (nature de la solution, durée et température) s'applique aux étapes ii₁') et ii₃'). De plus, la molécule comprenant une première portion P1 et une seconde portion P2 telle que précédemment définie et l'oligonucléotide à double structure tige-boucle est présent(e) dans la solution lors de la mise en contact respectivement à l'étape ii₁') et à l'étape ii₃') en une quantité comprise entre 1 aM et 1 mM, et notamment entre 100 aM et 1 µM. A titre d'exemples particuliers de concentrations utilisables, on peut citer une concentration de 10 pM, de 100 pM, de 1 nM, ou de 10 nM.

Suite à l'étape ii) ou ii₃') et préalablement à l'amplification LAMP ou suite à l'étape ii₁') et préalablement à l'étape ii₃'), il est nécessaire d'éliminer tous les éléments susceptibles de donner de faux positifs, comme, par exemple, des oligonucléotides à double structure tige-boucle ou des molécules à portions P1 et P2 ou des complexes de liaison non spécifiquement liés à l'analyte à détecter ou liés à des éléments non liés aux sondes fonctionnalisant la surface du support solide. Il s'agit là de l'étape ii₂'), iii) ou iii') du procédé selon la présente invention.

Toute technique permettant une telle élimination est utilisable dans le cadre de la présente invention. A titre d'exemple, on peut citer le lavage de la surface du support solide ou la séparation de la solution contenant des oligonucléotides à double structure tige-boucle ou des molécules à portions P1 et P2 ou des complexes de liaison et du support solide comme une séparation physique ou magnétique.

Lorsque cette étape d'élimination est mise en œuvre, la surface du support solide peut être soumise à au moins un rinçage qui est typiquement effectué avec une solution de rinçage constituée d'une solution aqueuse :
- conservant l'interaction analyte/oligonucléotide ou liant irréversiblement l'analyte et l'oligonucléotide (cas de l'étape iii)) ;
- conservant l'interaction analyte/complexe de liaison ou liant irréversiblement l'analyte et le complexe de liaison (cas de l'étape iii) et de l'étape iii')) ou
- conservant l'interaction analyte/molécule à portions P1 et P2 ou liant irréversiblement l'analyte et la molécule à portions P1 et P2 (cas de l'étape ii₂')).

Cette solution de rinçage peut également permettre d'éliminer les interactions non spécifiques. Elle peut comporter un ou plusieurs des composants suivants : un tampon comme un tampon Tris, phosphate, acétate ou borate ; des sels comme KCl, NaCl, (NH₄)₂SO₄, MgCl₂ ou CaCl₂ ; des détergents ou agents tensioactifs comme Tween^{®}, Triton^{®} ou dodécylsulfate de sodium (ou SDS) ; des agents dénaturants comme formamide ou diméthylsulfoxyde ; un solvant organique comme éthanol, méthanol ou acétonitrile ; et des agents pontants comme formaldéhyde ou glutaraldéhyde et un ou plusieurs élément(s) choisi(s) parmi un ADN génomique comme de l'ADN simple brin et notamment de l'ADN simple brin de sperme de saumon, du sérum, de l'albumine et un polymère de synthèse. Ce rinçage peut être répété deux fois, trois fois, cinq fois, 10 fois et voire 50 fois en utilisant, à chaque rinçage, une solution de rinçage identique ou différente. Typiquement, le rinçage est répété trois fois avec une solution comprenant du PBS et 0,1% de Tween^{®}. En variante, le rinçage est répété trois fois avec une solution saline contenant 0,02 M de PBS, 1,074 M de NaCl et 0,3% de détergent Tween^{®}20.

De plus, l'étape ii₂'), iii) ou iii') est réalisée à une température comprise entre 4°C et 100°C, notamment entre 15°C et 80°C, en particulier entre 30°C et 60°C et, plus particulièrement, à température de l'ordre de 40°C (i.e. 40°C ± 5°C) ou à température physiologique.

L'étape iv) ou iv') du procédé selon l'invention est l'amplification LAMP à deux amorces à proprement parler. Les deux seules amorces mises en œuvre lors de cette étape répondent respectivement à la formule (V) et à la formule (VI) ci-après :

5'-F1c-F2-3' (V)

5'-B1c-B2-3' (VI)

la portion B2 présentant une séquence nucléotidique complémentaire à la séquence nucléotidique de la portion B2c et les portions F1c, F2, B1c et B2c étant telles que précédemment définies.

Les amorces de formule (V) ou de formule (VI) sont respectivement appelées, dans l'amplification LAMP à quatre amorces, amorce FIP (pour « Forward Inner Primer ») et amorce BIP (pour « Backward Inner Primer »).

La température Tm de B2-B2c est notamment supérieure à la température utilisée lors de l'amplification et, en particulier, supérieure de 5°C à 7°C. L'homme du métier pourra trouver des informations additionnelles quant à ces portions dans **[1].**

La température Tm de F2-F2c est notamment supérieure à la température utilisée lors de l'amplification et, en particulier, supérieure de 5°C à 7°C. L'homme du métier pourra trouver des informations additionnelles quant à ces portions dans **[1].**

Il convient de noter que, dans l'étape iv) ou iv') du procédé selon la présente invention, l'analyte n'est pas utilisé comme une amorce de l'amplification LAMP.

L'étape iv) ou iv') du procédé selon la présente invention consiste à mettre en contact la surface sur laquelle est/sont immobilisée(s) une ou des sonde(s) spécifique(s) du ou des analytes à détecter avec un mélange réactionnel comprenant les amorces FIP et BIP et tout élément nécessaire à la production d'un produit d'amplification si l'oligonucléotide à double structure tige-boucle est présent.

Typiquement, le mélange réactionnel mis en œuvre lors de l'étape iv) ou iv') comprend un tampon approprié, comme, par exemple, un tampon phosphate ou un tampon Tris; des désoxyribonucléosides (dNTP), comme, un mélange équimolaire de dATP, dCTP, dGTP et dTTP et une enzyme catalysant l'amplification LAMP. Une telle enzyme est une polymérase présentant une activité de déplacement de brin élevée en plus d'une activité de réplication. A titre d'exemples particuliers de telles enzymes, on peut utiliser l'une des enzymes listées ci-dessous :
- Bst DNA polymerase et ses variantes telles que, par exemple, Bst2.0 DNA polymerase et Bst3.0 DNA polymerase,
- Bsm DNA polymerase,
- Bca (exo-) DNA polymerase,
- Klenow fragment of DNA polymerase I,
- Vent DNA polymerase,
- Vent(Exo-) DNA polymerase (exonuclease activity-free Vent DNA polymerase),
- DeepVent DNA polymerase,
- DeepVent(Exo-) DNA polymerase (DeepVent DNA polymerase sans activité exonucléase),
- 29 phage DNA polymerase,
- MS-2 phage DNA polymerase,
- Z-Taq DNA polymerase (Takara Shuzo), et
- KOD DNA polymerase (TOYOBO).

Le milieu réactionnel peut comprendre, en outre, des sels comme des sels de magnésium, des sels de manganèse et/ou sels d'ammonium ; des détergents, de la bétaïne et/ou des éléments utiles pour la détection et la quantification du produit d'amplification de l'oligonucléotide à double structure tige-boucle comme, par exemple, de la calcéine, un colorant intercalant comme, par exemple, iodure de propidium, SYTO 9, le vert SYBR et l'EvaGreen.

L'étape iv) ou iv') du procédé selon la présente invention est mise en œuvre pendant une période de temps et à une température suffisantes pour la production d'un produit d'amplification si l'oligonucléotide à double structure tige-boucle est présent. Avantageusement, cette étape est effectuée dans des conditions isothermes et notamment à une température comprise entre 15°C et 90°C, en particulier, comprise entre 50°C et 80°C et, plus particulièrement de l'ordre de 65°C (i.e. 65°C ± 5°C). La durée de l'étape iv) ou iv') du procédé selon la présente invention est comprise entre 1 min et 120 min, notamment entre 5 min et 90 min et, en particulier, entre 15 min et 60 min.

L'étape v) ou v') consiste à détecter et éventuellement à quantifier le produit d'amplification de l'oligonucléotide à double structure tige-boucle et donc à détecter et éventuellement à quantifier l'analyte auquel l'oligonucléotide à double structure tige-boucle est lié puisque l'accumulation du produit d'amplification est un indicateur de la présence de cet analyte immobilisé à la surface du support solide.

L'étape v) ou v') peut être réalisée simultanément à l'étape iv) ou iv') ou après cette dernière. L'homme du métier connaît différentes techniques pour réaliser cette détection. Cette dernière peut notamment être l'une quelconque des techniques décrites dans Becherer *et al,* 2020 **[6].**

Le produit d'amplification peut être détecté, lors de l'étape v) ou v') par mesure de la turbidité causée par le pyrophosphate de magnésium précipité en solution comme sous-produit de l'amplification. Cette mesure peut être réalisée à l'œil nu ou via un turbidimètre ou par imagerie sans lentille **[7].**

Le produit d'amplification peut être détecté, lors de l'étape v) ou v'), par une sonde pH permettant de suivre l'acidification du mélange réactionnel lors de l'amplification LAMP.

Le produit d'amplification peut être détecté, lors de l'étape v) ou v'), via une électrophorèse sur gel.

Le produit d'amplification peut être détecté, lors de l'étape v) ou v'), par détection de cristaux en plan par imagerie sans lentille comme décrit dans **[7].**

Le produit d'amplification peut également être détecté, lors de l'étape v) ou v'), par un test colorimétrique ou par mesure de fluorescence notamment lorsque la calcéine ou un colorant intercalant fluorescent est présent dans le mélange réactionnel.

Lorsque l'on souhaite réaliser une quantification d'au moins un analyte lors de l'étape v) ou v') du procédé selon la présente invention, cette quantification est réalisée par une comparaison avec une gamme étalon d'analyte à des concentrations connues. Cette quantification est une étape classique de la méthode ELISA. Cette quantification peut également mettre en œuvre des quantités connues d'analyte à détecter, ajoutées à l'échantillon liquide comme précédemment envisagé (échantillon liquide dopé).

D'autres caractéristiques et avantages de la présente invention apparaîtront encore à l'homme du métier à la lecture des exemples ci-dessous donnés à titre illustratif et non limitatif, en référence aux figures annexées.

### BRÈVE DESCRIPTION DES DESSINS

La Figure 1 présente la gamme de calibration LAMP d'un haltère tel que défini dans **[5]** (« Halt1 ») et de deux haltères selon la présente invention (« Halt2 » avec une entité de reconnaissance en 5' et « Halt3 » avec une entité de reconnaissance en 3'), les trois haltères comprenant une entité de reconnaissance de 18 bases.
La Figure 2 présente la gamme de calibration LAMP d'un haltère tel que défini dans **[5]** (« Halt4 ») et d'un haltère selon la présente invention (« Halt5 »), les deux haltères comprenant une entité de reconnaissance de 40 bases (n=2).
La Figure 3 présente le principe de détection directe d'un simple brin d'ADN complémentaire à une partie de l'haltère « DTBGE » selon l'invention.
La Figure 4 présente les courbes d'amplification de l'haltère « DTBGE » selon l'invention, hybridé sur le brin d'ADN complémentaire Thr1c immobilisé sur des billes magnétiques.
La Figure 5 présente la gamme de quantification de l'haltère « DTBGE » selon l'invention, hybridé sur le brin d'ADN complémentaire « Thr1c » immobilisé sur des billes magnétiques.
La Figure 6 présente le principe de la détection sandwich de brins d'ADN par l'haltère « DTBGE » selon l'invention (gauche) et contrôles positifs (droite).
La Figure 7 présente la gamme de quantification de l'oligonucléotide « Zip6Thrlc » avec différents haltères (« DTBGE » selon l'invention et « DTRC » tel que défini dans **[5]),** et contrôles positifs (« DTBGE contrôle positif» selon l'invention et « DTRC contrôle positif » tel que défini dans **[5]).**
La Figure 8 présente la gamme de quantification de l'oligonucléotide « Zip6Thrlc » (« Détection DTBGE (plasma) »), gamme de quantification des contrôles positifs (« Contrôle positif DTBGE (plasma) » et limites de détection basses (« Negative control (LOD basse) ») dans milieu complexe (plasma sanguin humain).
La Figure 9 présente le principe de la détection directe de molécules par reconnaissance avec l'haltère « DTBGE » selon l'invention.
La Figure 10 présente la gamme de quantification pour la détection de la thrombine en format direct, en duplicas expérimentaux (replica 1 et 2) et analytiques (courbes représentées avec des traits de même nature).
La Figure 11 présente la gamme de calibration pour la détection directe de thrombine avec haltère « DTBGE » selon la présente invention (données Figure 10).
La Figure 12 présente le principe de la détection de molécules par double sandwich aptamère et amplification LAMP.
La Figure 13 présente la gamme de quantification de la reconnaissance sandwich double aptamère de la thrombine, en duplicas expérimentaux (réplica 1 et 2) et analytiques (courbes représentées avec des traits de même nature).
La Figure 14 présente la gamme de quantification de la thrombine en tampon détectée en double sandwich aptamère avec l'haltère « DTBGE » selon l'invention.
La Figure 15 présente le principe de greffage indirect d'une entité de reconnaissance (aptamères ou anticorps) à l'extrémité de l'haltère selon la présente invention par hybridation d'ADN complémentaires.

### EXPOSÉ DÉTAILLÉ DE MODES DE RÉALISATION PARTICULIERS

### I. Comparaison LAMP avec haltère telle que décrite dans [5] et haltères selon la présente invention.

### 1.1. Protocole expérimental.

Plusieurs solutions d'haltères à des concentrations différentes ont été amplifiées selon le protocole d'amplification LAMP à deux amorces.

Le protocole consiste à ajouter 2 µL de solution d'haltère diluée dans un tampon contenant du PBS et du MgCl₂ (pH = 7,3) avec 18 µL de mix réactionnel comprenant :
- une enzyme catalysant l'amplification, Bst DNA Polymerase par exemple, avec son tampon d'amplification associé,
- un tampon d'amplification isotherme associé à l'enzyme, comme « isothermal amplification buffer (NEB) » par exemple,
- des désoxyribonucléosides triphosphates (dNTP),
- des sels de magnésium tels que MgCl₂,
- un colorant intercalant tel que l'EvaGreen,
- de la bétaïne et
- les amorces FIP et BIP telles que définies ci-après.

### 1.2. Entité de reconnaissance de 18 nucléotides.

L'entité de reconnaissance E1 mise en œuvre dans cet exemple comporte 18 bases. Dans l'haltère telle que définie dans **[5],** elle est insérée entre les portions F1 et B1c. Dans les deux cas conformes à la présente invention où l'entité de reconnaissance est ajoutée à l'extrémité (5' ou 3'), un bras espaceur de 10 bases thymine est ajouté avant. Un récapitulatif des séquences utilisées est présenté dans le Tableau 1 ci-après. Dans cet exemple, les haltères sont utilisés en une quantité variant de 10 nM à 10 pM dans les 2 µL de solutions.

**Tableau 1**

| **Nom** | **Construction** | **Séquence (5'-3')** | **Longueur** |
|---|---|---|---|
| **Halt1** (sonde au centre) [5] | 5'-F1c-F2-F1-E1-B1c-B2C-B1-3' | CGGATCCAAGACGCGGTTTATCGTGCAGTACGCCAACCT TTCTCAATAAACCGCGTCTTGGATCCGTGGTTGGTGTGGT TGGGACATCTCTGAGTTATATCTTTCCCCCCCTCACTGAC CCTCCTTCGGGGGAAAGATATAACTCAGAGATG | 152 |
| | | (SEQ ID NO: 1 dans le listage de séquences en annexe) | |
| **Halt2** (sonde en 5') | 5'-E1-10T-F1c-F2-F1-B1c-B2C-B1-3' | GGTTGGTGTGGTTGGTTTTTTTTTTCGGATCCAAGACGCG GTTTATCGTGCAGTACGCCAACCTTTCTCAATAAACCGCG TCTTGGATCCGTGACATCTCTGAGTTATATCTTTCCCCCCC TCACTGACCCTCCTTCGGGGGAAAGATATAACTCAGAGA TG | 162 |
| | | (SEQ ID NO: 2 dans le listage de séquences en annexe) | |
| **Halt3** (sonde en 3') | 5'-F1c-F2-F1-B1c-B2C-B1-10T-E1-3' | CGGATCCAAGACGCGGTTTATCGTGCAGTACGCCAACCT TTCTCAATAAACCGCGTCTTGGATCCGTGACATCTCTGAG TTATATCTTTCCCCCCCTCACTGACCCTCCTTCGGGGGAA AGATATAACTCAGAGATGTTTTTTTTTTGGTTGGTGTGGT TGG | 162 |
| | | (SEQ ID NO: 3 dans le listage de séquences en annexe) | |
| **Amorce FIP** | 5'-F1c-F2-3' | CGGATCCAAGACGCGGTTTATCGTGCAGTACGCCAACCT TTCTCA | 45 |
| | | (SEQ ID NO: 4 dans le listage de séquences en annexe) | |
| **Amorce BIP** | 5'-B1c-B2-3' | CATCTCTGAGTTATATCTTTCCCCCGAAGGAGGGTCAGTG AGG | 43 |
| | | (SEQ ID NO: 5 dans le listage de séquences en annexe) | |

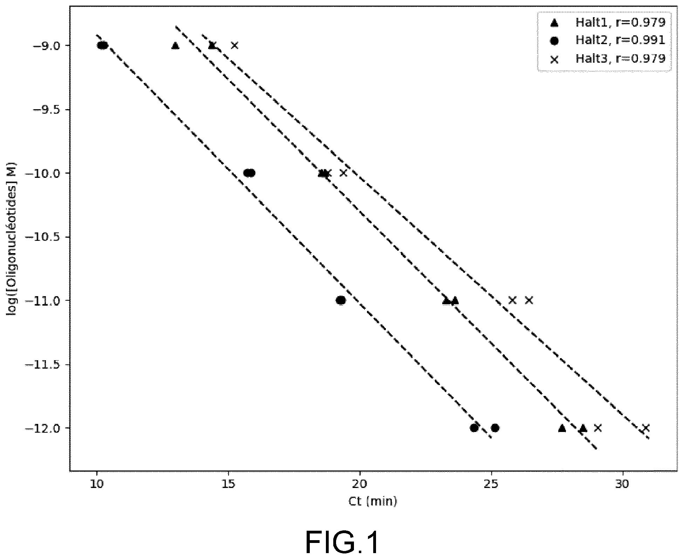

La gamme de calibration comparant les haltères décrits dans le Tableau 1 est présentée Figure 1.

Tout d'abord, on observe que l'amplification de l'haltère « Halt1 » est la plus lente et donc la moins optimale. Fait intéressant, lorsqu'une séquence est ajoutée en bout de chaîne (« Halt2 » et « Halt3 »), alors l'amplification est plus rapide. De plus, lors de l'ajout d'une séquence en bout de chaîne, la LAMP est plus efficace lorsque la séquence est ajoutée à l'extrémité 5', car elle est moins gênante dans le processus d'amplification, permettant notamment des ouvertures de brin plus faciles. Enfin, l'intégration de la sonde en extrémité de chaîne 5' est plus avantageuse par rapport aux configurations où la sonde était intégrée dans la séquence oligonucléotidique, comme c'était le cas dans **[5].**

Ainsi, l'haltère « Halt2 » contenant la sonde à l'extrémité 5' est particulièrement intéressant en termes d'application pour la reconnaissance moléculaire. Cet haltère présente le meilleur compromis entre rapidité de l'amplification LAMP et degrés de libertés apportés à l'entité de reconnaissance E pouvant contenir directement un aptamère ou bien servir de support pour greffer tout type de structure comprenant une entité de reconnaissance E par hybridation ou liaison covalente, et donc devient la séquence de choix pour les applications de détection.

### 1.3. Entité de reconnaissance de 40 nucléotides.

Comme présenté au point I.2 ci-dessus, il est possible d'ajouter une entité de reconnaissance E à l'extrémité 5' ou 3' de l'haltère, la configuration la plus intéressante pour les applications en détection correspondant à un ajout à l'extrémité 5'.

Deux nouveaux haltères ont été conçus avec une entité de reconnaissance E2 contenant 40 bases et présentés dans le Tableau 2 ci-après. Ces séquences sont amplifiables grâce au nouveau couple d'amorces FIP2 et BIP2.

**Tableau 2**

| **Nom** | **Construction** | **Séquence (5'-3')** | **Longueur** |
|---|---|---|---|
| **Halt4** | 5'-F1c-F2-F1-E2-B1c-B2C-B1-3' | AGAGCAGCAGAAGTGGCACAGGTGATTGTGAAGAAGAAG AGTGTGCCACTTCTGCTGCTCTCGTGCAGTACGCCAACCTT TCTCATGCGCTGCCCCTCTTATCAACCTGAAGAAGAGCAG GCAGTGAGGACAATCAGTTCT TGCTCTTCTTCAGGTTGA | 159 |
| | | (SEQ ID NO: 6 dans le listage de séquences en annexe) | |
| **Halt5** | 5'-E2-10T-F1c-F2-F1-Int-B1c-B2C-B1-3' | CGTGCAGTACGCCAACCTTTCTCATGCGCTGCCCCTCTTAT TTTTTTTTTAGAGCAGCAGAAGTGGCACAGGTGATTGTGA AGAAGAAGAGTGTGCCACTTCTGCTGCTCTTTATCAACCTG AAGAAGAGCAGGCAGTGAGGACAATCAGTTCT TGCTCTTCTTCAGGTTGA | 172 |
| | | (SEQ ID NO: 7 dans le listage de séquences en annexe) | |
| **Amorce FIP2** | 5'-F1c-F2-3' | AGAGCAGCAGAAGTGGCACAGGTGATTGTGAAGAAGAAG AG | 41 |
| | | (SEQ ID NO: 8 dans le listage de séquences en annexe) | |
| **Amorce BIP2** | 5'-B1c-B2-3' | TCAACCTGAAGAAGAGCAAGAACTGATTGTCCTCACTGCC (SEQ ID NO: 9 dans le listage de séquences en annexe) | 40 |

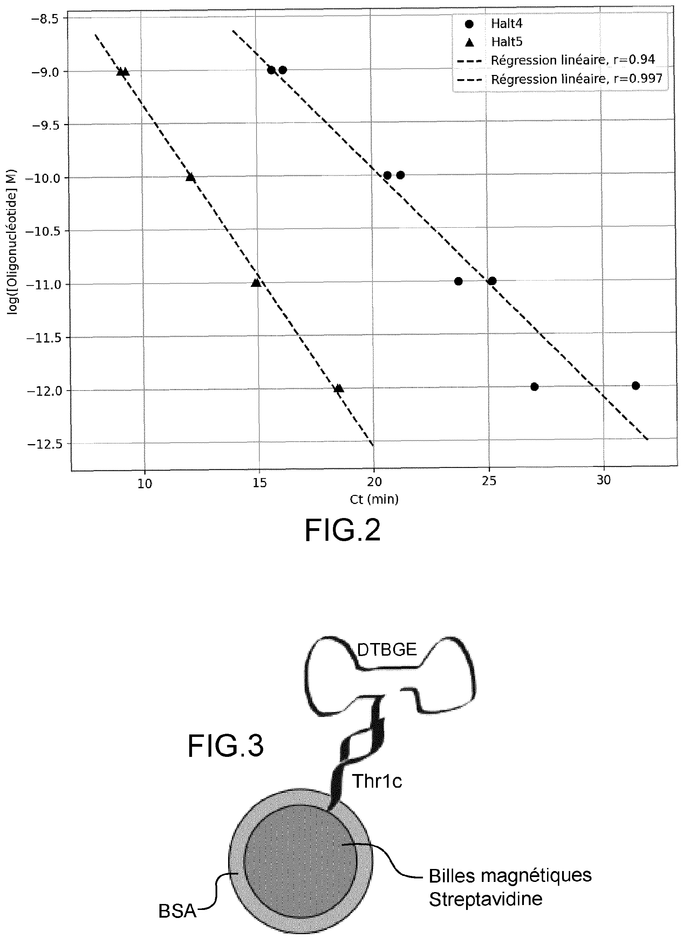

Ces deux haltères en une quantité variant de 10 nM à 10 pM dans les 2 µL de solution sont amplifiés selon le protocole de LAMP à deux amorces décrit précédemment. Ici, une enzyme plus rapide telle que Bst3.0 DNA Polymerase de chez New England Biolabs est utilisée du fait de la longueur importante des séquences à amplifier rendant l'amplification plus lente avec l'enzyme habituellement utilisée (i.e. Bst2.0 DNA Polymerase). Les courbes de calibration après amplification LAMP sont présentées Figure 2.

Ces résultats valident la fonctionnalité de ce nouvel haltère « Halt5 » nouvellement conçu contenant une nouvelle séquence en bout de chaîne qui pourra servir de sonde ou de support de sonde pour diverses applications. Cela montre aussi l'intérêt d'ajouter la séquence supplémentaire à l'extrémité de la chaîne et non à l'intérieur, par l'accélération de la LAMP pour l'haltère « Halt5 » par rapport à l'haltère « Halt4 ».

### II. Utilisation d'un haltère selon la présente invention en détection.

Dans cet exemple, l'utilisation de l'haltère « Halt2 » en détection est illustrée, suivant divers schémas innovants, car c'est l'haltère présentant le meilleur compromis entre longueur de séquence (rendement de synthèse chimique, acceptable et réaliste) et rapidité de l'amplification LAMP. De plus, cet haltère a été conçu afin de présenter une entité de reconnaissance correspondant à un aptamère « Thr1» reconnaissant la protéine thrombine **[8].** Dans ce qui suit, l'haltère « Halt2 » est dénommé « DTBGE » et l'entité de reconnaissance E1 est dénommée « Thr1 » (aptamère thrombine 1 selon **[8]).**

### II.1. Gamme de quantification d'oligonucléotides sur billes (direct).

Dans un premier temps, la détection de brins d'ADN a été réalisée et a permis de montrer que l'haltère selon la présente invention permettait de détecter un simple brin d'ADN lorsque celui-ci est complémentaire à la séquence placée à l'extrémité 5'. Deux protocoles de détection directs et indirects sont mis en place et testés avec l'haltère « DTBGE ».

Une gamme de différentes concentrations d'haltère mélangées avec des billes sur lesquelles sont greffées le brin d'ADN complémentaire à l'entité de reconnaissance « Thrlc » est réalisée. La solution contenant les billes et l'haltère est amplifiée après 3 lavages permettant d'éliminer les haltères ne s'étant pas accrochés à la cible. Le principe du protocole de détection est représenté Figure 3. Un contrôle négatif est réalisé en parallèle avec des billes contenant une séquence aléatoire « Zip6 » de séquence 5'-GACCGGTATGCGACCTGGTATGCG-3' (SEQ ID NO: 10 dans le listage de séquences en annexe) greffée, via une biotine sur les billes magnétiques streptavidine et non complémentaire à l'haltère DTBGE. « Zip6c » est de séquence 5'-CGCATACCAGGTCGCATACCGGTC-3' fonctionnalisée avec une fonction biotine en 5' (SEQ ID NO: 11 dans le listage de séquences en annexe).

Le protocole de détection est alors le suivant :
- incubation du brin d'ADN biotinylé « Thrlc » de séquence 5'-CCAACCACACCAACC-3' fonctionnalisée avec une fonction biotine en 5' (SEQ ID NO: 12 dans le listage de séquences en annexe) avec les billes magnétiques streptavidine à 25°C pendant 10 min puis rinçage à l'aide d'un aimant et d'un tampon de rinçage comprenant du PBS, 1,074 M de NaCl et 0,3% de Tween^{®}20 (ci-après « tampon de rinçage Tr »),
- blocage des billes magnétiques pendant 30 min à l'aide d'une solution d'hybridation contenant de la BSA (pour « Bovine Serum Albumin »),
- incubation de différentes concentrations d'haltère (1 nM - 100 pM) à 25°C pendant 3 h, puis 3 rinçages à 40°C avec le tampon de rinçage Tr,
- ajout de la solution LAMP contenant 2,4 µM de chacune des amorces FIP et BIP et l'enzyme nécessaire à l'amplification à savoir 0,4 U/ml de Enzyme Bst2.0 DNA Polymerase (NEB) (ci-après « solution mix LAMP »).

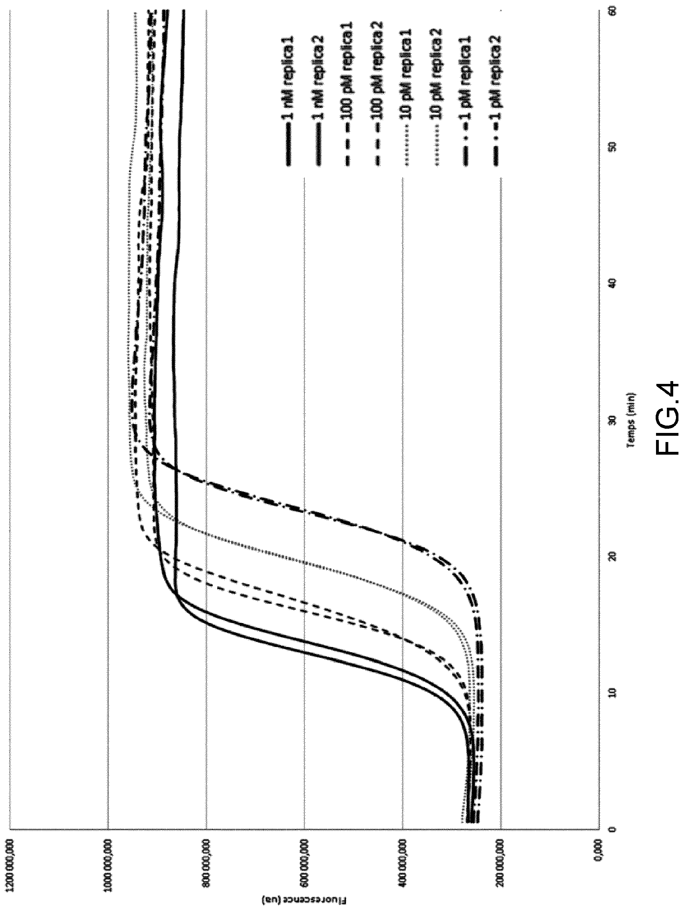

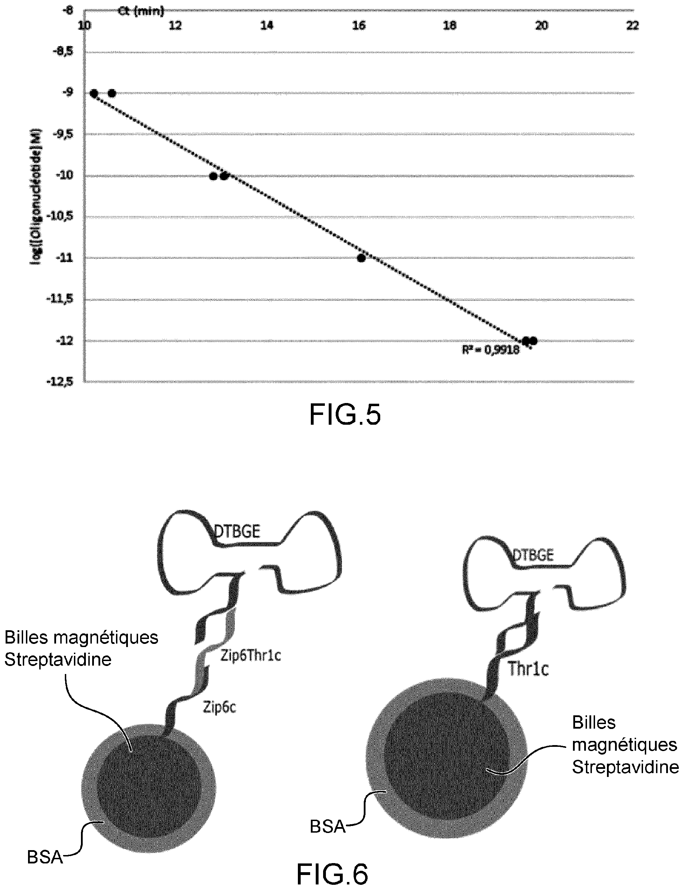

Ce protocole permet d'obtenir une gamme d'amplification de l'haltère lorsque son extrémité 5' s'hybride avec le brin complémentaire. Les courbes d'amplification obtenues et la gamme de calibration associée sont exposées Figure 4 et Figure 5 respectivement.

Des contrôles négatifs sont également effectués avec des billes sur lesquelles est immobilisé un brin d'ADN non complémentaire à l'haltère (« Zip6c »). Ces contrôles sont mis en solution avec les réactifs LAMP et permettent d'évaluer la part du signal non spécifique. Les signaux correspondant au contrôle négatif se révèlent autour de 17 min, ce qui permet d'envisager une quantification de l'haltère jusqu'à une concentration de 10 pM.

### II.2. Gamme de quantification d'oligonucléotides en format sandwich (indirect).

Une détection de brin d'ADN en format sandwich est effectuée. Le principe de la détection est exposé Figure 6. Ici, la gamme de quantification d'oligonucléotide sur billes présentée précédemment sert de contrôle positif à la détection indirecte.

Le protocole mis en place est le suivant :
- incubation du brin d'ADN biotinylé « Zip6c » avec les billes magnétiques streptavidine à 25°C pendant 10 min puis rinçage à l'aide d'un aimant et du tampon de rinçage Tr,
- blocage des billes magnétiques pendant 30 min à l'aide d'une solution d'hybridation contenant de la BSA,
- incubation de différentes concentrations de cible « Zip6Thr1c » (1 nM - 1 pM) à 37°C pendant 30 min puis 3 rinçages avec le tampon de rinçage Tr. La cible « Zip6Thrlc » est de séquence 5'- CCAACCACACCAACCGACCGGTATGCGACCTGGTATGCG-3' (SEQ ID NO: 13 dans le listage de séquences en annexe),
- ajout de la solution d'haltère à 100 pM et incubation à 37°C pendant 3 h, puis 3 rinçages à 40°C avec le tampon de rinçage Tr,
- ajout de la solution mix LAMP.

Le protocole pour les contrôles positifs est similaire mais le brin « Thrlc » complémentaire à l'haltère est immobilisé sur les sondes, aucune cible « Zip6Thrlc » n'est ajouté, et enfin 4 concentrations d'haltère différentes sont ajoutées (1 nM - 1 pM).

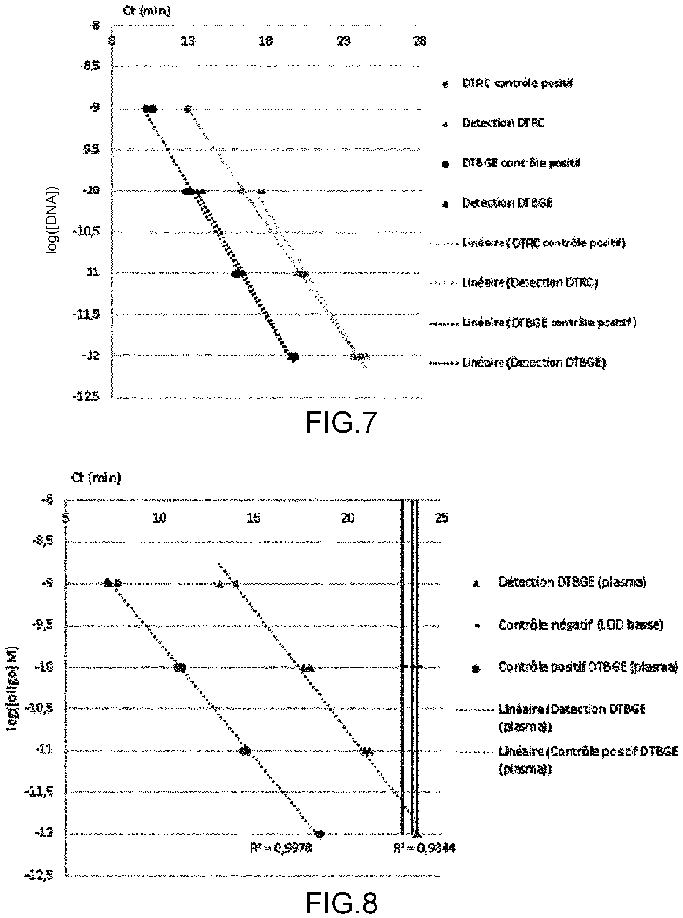

Le même protocole est effectué avec l'haltère « Halt1 » (ici nommé « DTRC ») tel que défini dans **[5]** pour évaluer la performance de l'haltère selon la présente invention. Les résultats pour la gamme de quantification sont présentés Figure 7.

Ces derniers résultats valident la détection d'un simple brin d'ADN avec l'haltère « DTBGE » et son avantage par rapport à l'haltère « DTRC ». La gamme sur billes correspond aux contrôles positifs et permet de valider la méthode par une détection. Enfin, la détection sandwich avec l'haltère « DTBGE » présente l'avantage majeur d'être plus rapide (moins de 20 min) que l'haltère « DTRC » tel que défini dans **[5].**

Dans un second temps, la détection sandwich est effectuée dans un milieu complexe (plasma sanguin humain) afin de se rapprocher des conditions expérimentales sur échantillons cliniques. Les résultats sont présentés Figure 8. On obtient deux gammes linéaires : les contrôles positifs (haltères + billes) et la gamme de détection sandwich. Les 4 points correspondant aux contrôles négatifs apparaissent au niveau du dernier point de la gamme de détection, ce qui donne une limite de détection basse de 1 pM pour la détection d'oligonucléotides avec l'haltère selon l'invention.

La méthode utilisant un haltère selon l'invention permet donc de détecter et quantifier un oligonucléotide en milieu complexe jusqu'à une concentration de 1 pM en moins de 30 min d'amplification.

### 11.3. Gamme de quantification de protéines sur billes (direct).

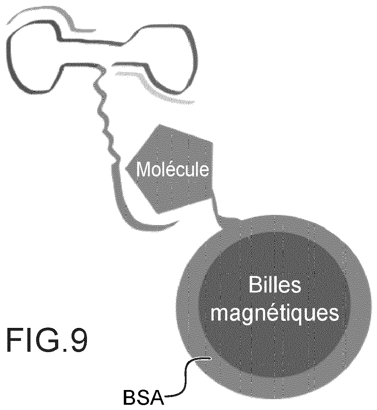

Un mode de détection direct consiste à fixer une molécule d'intérêt sur des billes au préalable par liaison covalente, et l'haltère vient détecter la molécule grâce à l'aptamère spécifique situé à l'extrémité 5' de l'haltère « DTBGE ». Le schéma de ce type de détection est présenté Figure 9.

Afin de valider la détection de protéine, ce protocole est réalisé avec l'haltère « DTBGE » qui contient l'aptamère « Thr1» à l'extrémité 5' spécifique de la thrombine, immobilisée préalablement sur des billes magnétiques.

Le protocole mis en œuvre est le suivant :
- préparation de différentes solutions de billes contenant différentes concentrations de thrombine (100 nM - 1 nM),
- blocage des billes magnétiques pendant 30 min à l'aide d'une solution de BSA,
- incubation avec la solution d'haltère « DTBGE » à 100 pM sous agitation à 25°C pendant 2 h,
- ajout de la solution mix LAMP.

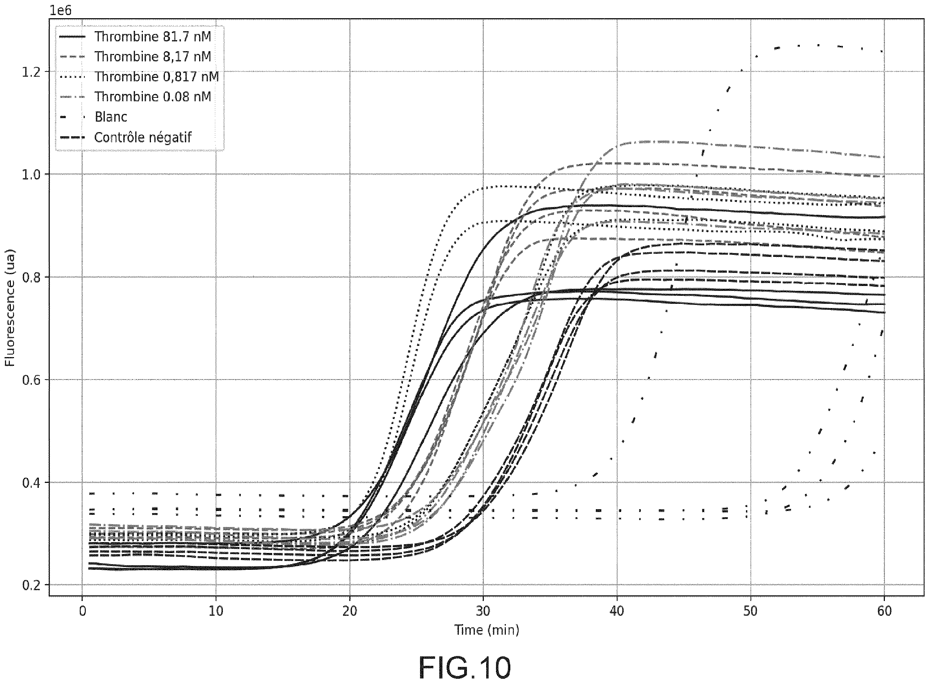

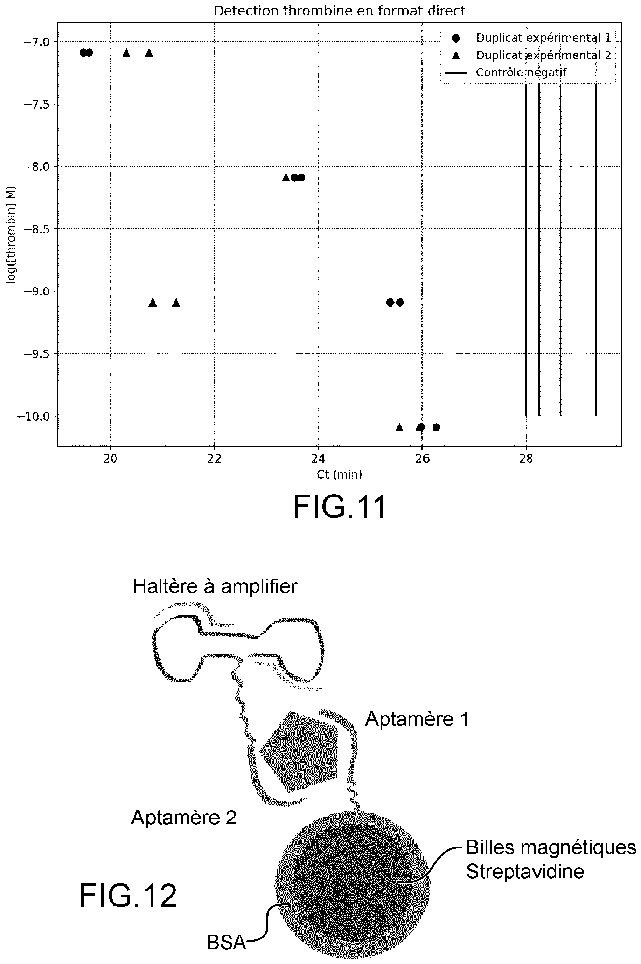

Les résultats de l'amplification sont présentés Figure 10. La Figure 11 est une preuve de concept d'une possible quantification de la thrombine jusqu'à l'échelle nanomolaire en moins de 30 min, par reconnaissance de l'aptamère placé en bout de chaîne de l'haltère et spécifique de la molécule à reconnaître.

Cette expérience valide donc l'utilisation de l'haltère « DTBGE » selon l'invention pour reconnaître une protéine dans un format de détection direct. Cependant, pour des applications de détection dans des échantillons complexes, il est intéressant de pouvoir détecter des molécules en format sandwich et de manière spécifique. Le point II.4. ci-après s'intéresse alors à la détection en format sandwich grâce à l'haltère « DTBGE » selon l'invention.

### II.4. Gamme de quantification de protéines en format sandwich (indirect).

Suite à la validation de l'utilisation de l'haltère selon l'invention pour la reconnaissance de molécules en format direct, un double sandwich aptamères peut être envisagé et permettre de détecter des molécules de manière indirecte dans un échantillon complexe. Le principe d'une telle détection est exposé Figure 12.

Le protocole utilisé pour la détection de la molécule thrombine, en mettant en œuvre deux aptamères en reconnaissance sandwich issus de la littérature [6] est le suivant :
- immobilisation des sondes oligonucléotiques « Thr2 » de séquence 5'-AGTCCGTGGTAGGGCAGGTTGGGGTGACT-3' fonctionnalisée avec une fonction biotine en 5' (SEQ ID NO: 14 dans le listage de séquences en annexe) sur les billes magnétiques,
- blocage des billes magnétique à la BSA pendant 30 min,
- incubation de différentes concentrations de la cible thrombine sous agitation à 25°C pendant 2 h,
- incubation avec l'haltère « DTBGE » à 100 pM sous agitation à 25°C pendant 30 min,
- ajout de la solution mix LAMP.

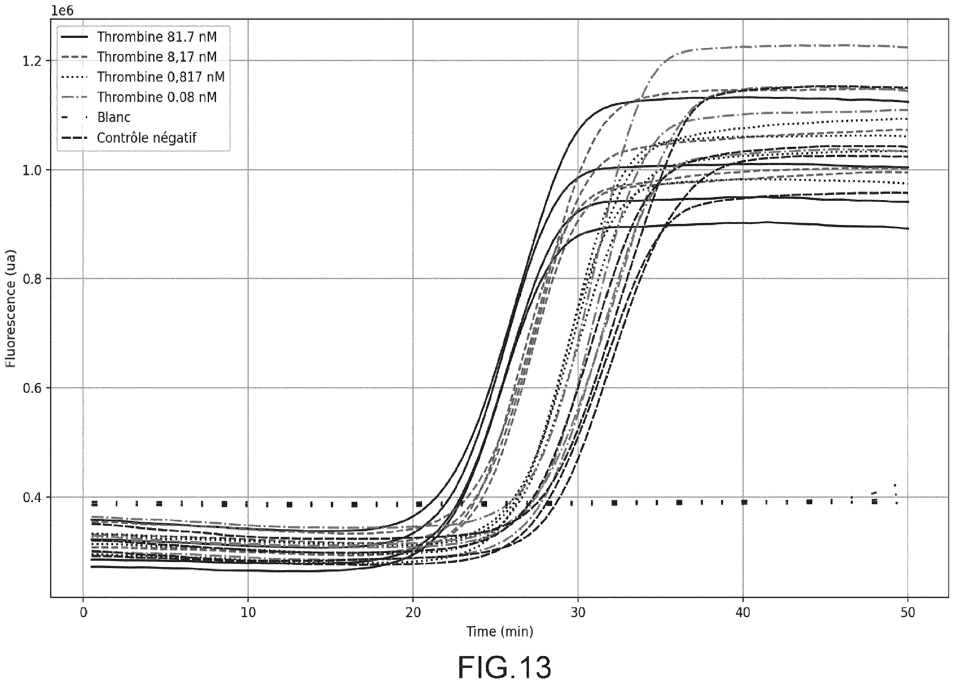

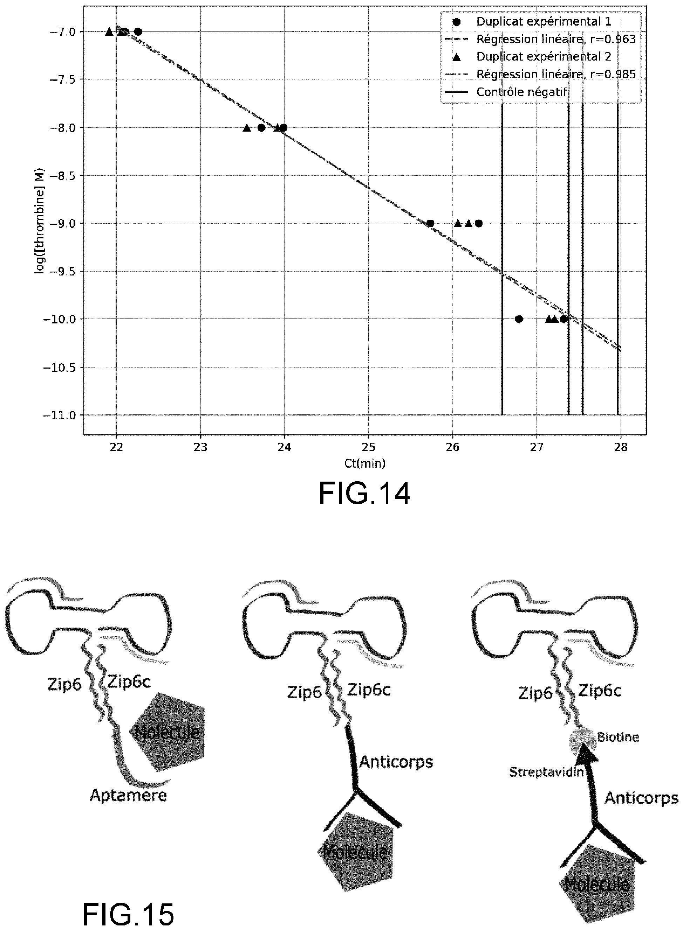

Ces derniers résultats présentés Figure 13 et Figure 14 montrent la preuve de concept de la détection de la thrombine avec un double sandwich aptamère et une possible quantification jusqu'à 1 nM de thrombine en moins de 30 min d'amplification avec l'haltère « DTBGE » selon la présente invention. Cette méthode a l'avantage principal de s'affranchir totalement de l'utilisation d'anticorps et de permettre de détecter des petites quantités grâce à la sensibilité donnée par la LAMP.

### III. Autre variant d'un haltère selon la présente invention.

L'haltère selon la présente invention a l'avantage majeur d'être générique et plusieurs schémas de détection peuvent être envisagés, dont plusieurs ont été détaillés au point II ci-dessus.

En variante, l'haltère selon la présente invention peut présenter une séquence générique de type « Zip » intégrée en extrémité de chaîne 5' servant de support pour hybrider un autre brin oligonucléotidique qui pourra alors contenir divers types d'entités de reconnaissance, sans gêner l'amplification LAMP. Ce complexe forme alors une sonde de reconnaissance composée de deux parties liées par hybridation d'ADN qui est une liaison chimique non covalente.

On peut envisager trois exemples d'un tel variant illustrés à la Figure 15 :
- une molécule comprenant une portion P1 du type séquence oligonucléotidique complémentaire et une portion P2 du type aptamère qui vient s'hybrider sur l'entité E de l'haltère. L'aptamère peut détecter une molécule. Cet exemple permet notamment d'intégrer des aptamères ARN impossible avec les haltères tel que décrits dans l'art antérieur,
- une molécule comprenant une portion P1 du type séquence oligonucléotidique complémentaire et une portion P2 du type anticorps qui vient s'hybrider sur l'entité E de l'haltère. Cet anticorps sert alors de détection pour la molécule d'intérêt et est couplé de manière covalente ou non à l'haltère amplifiable par LAMP à deux amorces, et
- une molécule comprenant une portion P1 du type séquence oligonucléotidique complémentaire et une portion P2 du type anticorps qui vient s'hybrider sur l'entité E de l'haltère, la portion P1 et la portion P2 étant couplées l'une à l'autre par liaison biotine-streptavidine. Cet anticorps sert alors de détection pour la molécule d'intérêt et est couplé à l'haltère amplifiable par LAMP à deux amorces.

Cette méthode est la plus générique possible car elle s'adapte à un large nombre de molécules à partir d'un seul haltère. L'ensemble de ces différentes molécules détecte des cibles de manière sensible grâce à l'amplification LAMP.

### Références bibliographiques

[1] Brevet US 6,410,278 publié le 25 juin 2002.
[2] Pourhassan-Moghaddam et al, 2013, « Protein détection through différent platforms of immuno-loop-mediated isothermal amplification », Nanoscale Research Letters, vol. 8:485, pages 1-11.
[3] Du et al, 2016, « A ligation-based loop-mediated isothermal amplification (ligation-LAMP) strategy for highly selective microRNA détection », Chem. Commun., vol. 52, pages 12721-12724.
[4] Demande de brevet CN 106148549 publiée le 23 novembre 2016.
[5] Demande de brevet EP 3878971 publiée le 15 septembre 2021.
[6] Becherer et al, 2020, « Loop-mediated isothermal amplification (LAMP) - review and classification of methods for sequence-specific détection », vol. 12, pages 717-746.
[7] Demande de brevet EP 3363913 publiée le 22 août 2018.
[8] Daniel et al, 2013, « Real-time monitoring of thrombin interactions with its aptamers: Insights into the sandwich complex formation », Biosens. Bioelectron., vol. 40, pages 186-192.

## Revendications

1. Oligonucléotide à double structure tige-boucle objet répondant à la formule (II) ou (III) suivante :
S-F1c-F2-F0'-F1-Int-B1c-B0'-B2c-B1 (II)
F1c-F2-F0'-F1-Int-B1c-B0'-B2c-B1-S (III)
dans lesquelles
la portion F1c présente une séquence nucléotidique complémentaire à la séquence nucléotidique de la portion F1 moyennant quoi la portion F1c et la portion F1 s'hybrident pour former la tige de la première structure tige-boucle, ladite portion F1 et ladite portion F1c comprenant, chacune, de 10 à 35 nucléotides,
F0' séparant la portion F2 et la portion F1 est soit une liaison covalente, soit une portion comprenant au moins un nucléotide,
la portion F2-F0' forme la boucle de la première structure tige-boucle, désignée ci-après boucle F0,
la portion B1 présente une séquence nucléotidique complémentaire à la séquence nucléotidique de la portion B1c moyennant quoi la portion B1 et la portion B1c s'hybrident pour former la tige de la seconde structure tige-boucle,
B0' séparant la portion B1c et la portion B2c est soit une liaison covalente, soit une portion comprenant au moins un nucléotide,
la portion B0'-B2c forme la boucle de la seconde structure tige-boucle, désignée ci-après boucle B0,
Int séparant la portion F1 et la portion B1c est soit une liaison covalente, soit une portion comprenant au moins un nucléotide, et
S représente une structure comprenant au moins une entité E apte à se lier, directement ou indirectement, à un analyte d'intérêt.

2. Oligonucléotide selon la revendication 1, **caractérisé en ce que**
- ladite portion B1 et ladite portion B1c comprennent, chacune, de 10 à 35 nucléotides, et/ou
- ladite portion F2 comprend de 8 à 30 nucléotides et/ou
- ladite portion B2c comprend de 8 à 35 nucléotides.

3. Oligonucléotide selon la revendication 1 ou 2, **caractérisé en ce que**
- lorsque F0' est une portion comprenant au moins un nucléotide, ladite portion F0' est telle que ladite boucle F0 formée par la portion F2-F0' comprend de 10 à 70 nucléotides, et/ou
- lorsque B0' est une portion comprenant au moins un nucléotide, ladite portion B0' est telle que la boucle B0 formée par la portion B0'-B2c comprend de 10 à 70 nucléotides.

4. Oligonucléotide selon l'une quelconque des revendications 1 à 3, **caractérisé en ce que** ladite structure S comprend, en plus de ladite au moins une entité E, au moins un autre élément permettant de lier ladite au moins une entité E à l'extrémité 5' de la portion F1c de l'oligonucléotide de formule (II) ou à l'extrémité 3' de la portion B1 de l'oligonucléotide de formule (III).

5. Oligonucléotide selon l'une quelconque des revendications 1 à 4, **caractérisé en ce que** ladite au moins une entité E est apte à se lier directement à au moins un analyte d'intérêt.

6. Oligonucléotide selon la revendication 5, **caractérisé en ce que** ladite au moins une entité E est choisie dans le groupe constitué par un glucide ; un peptide ; un antigène ; un épitope ; une protéine ; une glycoprotéine ; une enzyme ; un substrat enzymatique; un récepteur membranaire ou nucléaire; un agoniste ou un antagoniste d'un récepteur membranaire ou nucléaire; une toxine; un anticorps polyclonal ou monoclonal ; un fragment d'anticorps ; une molécule nucléotidique et un aptamère.

7. Oligonucléotide selon l'une quelconque des revendications 1 à 4, **caractérisé en ce que** ladite au moins une entité E est apte à se lier indirectement à au moins un analyte d'intérêt.

8. Complexe non-covalent, formé par un oligonucléotide selon la revendication 7 et une molécule comprenant une première portion P1 apte à se lier à ladite au moins une entité E dudit oligonucléotide et une seconde portion P2 apte à se lier à au moins un analyte d'intérêt.

9. Utilisation d'un oligonucléotide à double structure tige-boucle selon l'une quelconque des revendications 1 à 7 ou un complexe non covalent selon la revendication 8 pour détecter et éventuellement quantifier au moins un analyte éventuellement présent dans un échantillon liquide.

10. Procédé pour détecter et éventuellement quantifier au moins un analyte éventuellement présent dans un échantillon liquide, comprenant les étapes suivantes :
i) mettre en contact ledit échantillon liquide avec la surface d'un support solide comprenant au moins une zone active sur laquelle au moins une sonde apte à lier ledit au moins un analyte est immobilisée ;
ii) mettre en contact ladite surface avec une solution contenant soit au moins un oligonucléotide selon la revendication 5 ou 6, soit un complexe selon la revendication 8, ledit oligonucléotide et ledit complexe ayant été préparés préalablement à ladite mise en contact ;
iii) éliminer l'excès d'oligonucléotides ou l'excès de complexes n'ayant pas réagi lors de la mise en contact de l'étape ii) ;
iv) mettre en contact ladite surface avec deux amorces d'amplification isotherme médiée par des boucles dans des conditions permettant l'amplification dudit oligonucléotide ;
v) détecter et éventuellement quantifier le produit de l'amplification dudit oligonucléotide.

11. Procédé pour détecter et éventuellement quantifier un analyte éventuellement présent dans un échantillon liquide, comprenant les étapes suivantes :
i') mettre en contact ledit échantillon liquide avec la surface d'un support solide comprenant au moins une zone active sur laquelle au moins une sonde apte à lier ledit au moins un analyte est immobilisée ;
ii₁') mettre en contact ladite surface avec une solution contenant au moins une molécule comprenant une première portion P1 apte à se lier à l'entité E présente dans la structure de l'oligonucléotide selon la revendication 7 et une seconde portion P2 apte à se lier audit au moins un analyte d'intérêt ;
ii₂') éliminer l'excès de molécules n'ayant pas réagi lors de la mise en contact de l'étape ii₁') ;
ii₃') mettre en contact ladite surface avec une solution contenant au moins un oligonucléotide selon la revendication 7, ledit oligonucléotide ayant été synthétisé préalablement à ladite mise en contact ;
iii') éliminer l'excès d'oligonucléotides n'ayant pas réagi lors de la mise en contact de l'étape ii₃') ;
iv') mettre en contact ladite surface avec deux amorces d'amplification isotherme médiée par des boucles dans des conditions permettant l'amplification dudit oligonucléotide ;
v') détecter et éventuellement quantifier le produit de l'amplification dudit oligonucléotide.

12. Procédé selon la revendication 10 ou 11, **caractérisé en ce que** ledit produit d'amplification est détecté, lors de ladite étape v) ou v'), par mesure de la turbidité, par imagerie sans lentille, par une sonde pH, via une électrophorèse sur gel, par un test colorimétrique ou par mesure de fluorescence.
